# EUROPEAN PATENT APPLICATION

(11) **EP 1 360 962 A2**
(43) Date of publication of application: **12.11.2003**
(21) Application number: 03006878.7
(22) Date of filing: 19.10.1993
(51) Int. Cl.: A61K 38/21

(54) **Interferon tau composition and methods of use**

(30) Priority: 30.10.1992 US 969890
(62) Divisional of application: 93924958.7
(71) Applicant: UNIVERSITY OF FLORIDA, Gainesville, Florida 32611-0330 (US); THE WOMEN'S RESEARCH INSTITUTE, Wichita, KS 67214 (US)
(72) Inventor: Bazer, Fuller Warren, Texas A&M Univ., College Station, TX 77843-2471 (US); Johnson, Howard Marcellus, Univ. of Florida, Gainesville, FL 32611-0330 (US); Pontzer, Carol Hanlon, University of Maryland, College Park, MD 20742 (US); Ott, Troy Lee, Texas A&M Univ., College Station, TX 77843-2471 (US); van Heeke, Gino, Horsham, West Sussex (GB); Imakawa, Kazuhiko, Lab. of Animal Breeding, Bunkyo-ku, Tokyo 113-8657 (JP)
(74) Representative: VOSSIUS & PARTNER

(57) **Abstract**

The present invention describes a composition comprising interferon-τ and its use for the manufacture of a medicament.

SEQUENCE LISTING

(1) GENERAL INFORMATION:

(i) APPLICANT: University of Florida
The Women's Research Institute

(ii) TITLE OF INVENTION: Interferon Tau Compositions and
Methods of Use

(iii) NUMBER OF SEQUENCES: 20

(iv) CORRESPONDENCE ADDRESS:
(A) ADDRESSEE: Law Offices of Peter J. Dehlinger
(B) STREET: 350 Cambridge Ave., Suite 300
(C) CITY: Palo Alto
(D) STATE: CA
(E) COUNTRY: USA
(F) ZIP: 94306

(v) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.25

(vii) PRIOR APPLICATION DATA:
(A) APPLICATION NUMBER: US 07/969,890
(B) FILING DATE: 30-OCT-1992

(viii) ATTORNEY/AGENT INFORMATION:
(A) NAME: Fabian, Gary R.
(B) REGISTRATION NUMBER: 33,875
(C) REFERENCE/DOCKET NUMBER: 5600-0001.41

(ix) TELECOMMUNICATION INFORMATION:
(A) TELEPHONE: 415-324-0880

(B) TELEFAX: 415-324-0960

(2) INFORMATION FOR SEQ ID NO:1:

(i) SEQUENCE CHARACTERISTICS:
(A) LENGTH: 518 base pairs
(B) TYPE: nucleic acid
(C) STRANDEDNESS: double
(D) TOPOLOGY: circular

(ii) MOLECULE TYPE: DNA

(iii) HYPOTHETICAL: NO

(iv) ANTI-SENSE: NO

(vi) ORIGINAL SOURCE:
(A) ORGANISM: Ovis aries
(B) STRAIN: Domestic
(D) DEVELOPMENTAL STAGE: Blastula (blastocyst)
(F) TISSUE TYPE: Trophectoderm
(G) CELL TYPE: Mononuclear trophectoderm cells

(vii) IMMEDIATE SOURCE:
(B) CLONE: oTP-1a

(viii) POSITION IN GENOME:
(C) UNITS: bp

(ix) FEATURE:
(A) NAME/KEY: CDS
(B) LOCATION: 1..518

(x) PUBLICATION INFORMATION:
(A) AUTHORS: Ott, Troy L
Van Heeke, Gino
Johnson, Howard M
Bazer, Fuller W
(B) TITLE: Cloning and Expression in Saccharomyces
cerevisiae of a Synthetic Gene for the Type I
Trophoblast Interferon Ovine Trophoblast

Protein-1:Purification and Antiviral Activity
(C) JOURNAL: J. Interferon Res.
(D) VOLUME: 11
(F) PAGES: 357-364
(G) DATE: 1991
(K) RELEVANT RESIDUES IN SEQ ID NO:1: FROM 1 TO 518

(xi) SEQUENCE DESCRIPTION: SEQ ID NO:1:

TGC TAC CTG TCG CGA AAA CTG ATG CTG GAC GCT CGA GAA AAT TTA AAA 48
Cys Tyr Leu Ser Arg Lys Leu Met Leu Asp Ala Arg Glu Asn Leu Lys
1               5                   10                  15

CTG CTG GAC CGT ATG AAT CGA TTG TCT CCG CAC AGC TGC CTG CAA GAC 96
Leu Leu Asp Arg Met Asn Arg Leu Ser Pro His Ser Cys Leu Gln Asp
            20                  25                  30

CGG AAA GAC TTC GGT CTG CCG CAG GAA ATG GTT GAA GGT GAC CAA CTG 144
Arg Lys Asp Phe Gly Leu Pro Gln Glu Met Val Glu Gly Asp Gln Leu
        35                  40                  45

CAA AAA GAC CAA GCT TTC CCG GTA CTG TAT GAA ATG CTG CAG CAG TCT 192
Gln Lys Asp Gln Ala Phe Pro Val Leu Tyr Glu Met Leu Gln Gln Ser
    50                  55                  60

TTC AAC CTG TTC TAC ACT GAA CAT TCT TCG GCC GCT TGG GAC ACT ACT 240
Phe Asn Leu Phe Tyr Thr Glu His Ser Ser Ala Ala Trp Asp Thr Thr
65                  70                  75                  80

CTT CTA GAA CAA CTG TGC ACT GGT CTG CAA CAG CAA CTG GAC CAT CTG 288
Leu Leu Glu Gln Leu Cys Thr Gly Leu Gln Gln Gln Leu Asp His Leu
                85                  90                  95

GAC ACT TGC CGT GGC CAG GTT ATG GGT GAA GAA GAC TCT GAA CTG GGT 336
Asp Thr Cys Arg Gly Gln Val Met Gly Glu Glu Asp Ser Glu Leu Gly
            100                 105                 110

AAC ATG GAT CCG ATC GTT ACT GTT AAA AAA TAT TTC CAG GGT ATC TAC 384
Asn Met Asp Pro Ile Val Thr Val Lys Lys Tyr Phe Gln Gly Ile Tyr
        115                 120                 125

GAC TAC CTG CAG GAA AAA GGT TAC TCT GAC TGC GCT TGG GAA ATC GTA 432
Asp Tyr Leu Gln Glu Lys Gly Tyr Ser Asp Cys Ala Trp Glu Ile Val
    130                 135                 140

CGC GTT GAA ATG ATG CGG GCC CTG ACT GTG TCG ACT ACT CTG CAA AAA 480
Arg Val Glu Met Met Arg Ala Leu Thr Val Ser Thr Thr Leu Gln Lys
145                 150                 155                 160

CGG TTA ACT AAA ATG GGT GGT GAC CTG AAT TCT CCG 518
Arg Leu Thr Lys Met Gly Gly Asp Leu Asn Ser Pro
                165                 170

(2) INFORMATION FOR SEQ ID NO:2:

(i) SEQUENCE CHARACTERISTICS:
(A) LENGTH: 172 amino acids
(B) TYPE: amino acid
(D) TOPOLOGY: linear

(ii) MOLECULE TYPE: protein

(xi) SEQUENCE DESCRIPTION: SEQ ID NO:2:

Cys Tyr Leu Ser Arg Lys Leu Met Leu Asp Ala Arg Glu Asn Leu Lys
1               5                   10                  15

Leu Leu Asp Arg Met Asn Arg Leu Ser Pro His Ser Cys Leu Gln Asp
            20                  25                  30

Arg Lys Asp Phe Gly Leu Pro Gln Glu Met Val Glu Gly Asp Gln Leu
        35                  40                  45

Gln Lys Asp Gln Ala Phe Pro Val Leu Tyr Glu Met Leu Gln Gln Ser
    50                  55                  60

Phe Asn Leu Phe Tyr Thr Glu His Ser Ser Ala Ala Trp Asp Thr Thr
65                  70                  75                  80

92

Leu Leu Glu Gln Leu Cys Thr Gly Leu Gln Gln Gln Leu Asp His Leu
                85                  90                  95

Asp Thr Cys Arg Gly Gln Val Met Gly Glu Glu Asp Ser Glu Leu Gly
            100                 105                 110

Asn Met Asp Pro Ile Val Thr Val Lys Lys Tyr Phe Gln Gly Ile Tyr
        115                 120                 125

Asp Tyr Leu Gln Glu Lys Gly Tyr Ser Asp Cys Ala Trp Glu Ile Val
    130                 135                 140

Arg Val Glu Met Met Arg Ala Leu Thr Val Ser Thr Thr Leu Gln Lys
145                 150                 155                 160

Arg Leu Thr Lys Met Gly Gly Asp Leu Asn Ser Pro
                165                 170

(2) INFORMATION FOR SEQ ID NO:3:

(i) SEQUENCE CHARACTERISTICS:
(A) LENGTH: 516 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO:3:

TGTGACTTGT CTCAAAACCA CGTTTTGGTT GGTAGAAAGA ACTTAAGACT ACTAGACGAA 60

ATGAGACGTC TATCTCCACA CTTCTGTCTA CAAGACAGAA AGGACTTCGC TTTGCCTCAG 120

GAAATGGTTG AAGGTGGCCA ACTACAAGAA GCTCAAGCGA TATCTGTTTT GCACGAAATG 180

TTGCAACAAA GCTTCAACTT GTTCCACACC GAACACTCTT CGGCCGCTTG GGACACCACC 240

TTGTTGGAAC CATGTAGAAC CGGTTTGCAC CAACAATTGG ACAACTTGGA TGCATGTTTG 300

GGTCAAGTTA TGGGTGAAGA AGACTCTGCT CTCGGGAGAA CCGGTCCAAC GCTAGCTTTG 360

AAGAGATACT TCCAAGGTAT CCACGTTTAC TTGAAGGAAA AGGGTTACTC TGACTGTGCT 420

TGGGAAACCG TGCGTCTAGA AATCATGCGT AGCTTCTCTT CTTTGATCAG CTTGCAAGAA 480

AGATTACGTA TGATGGACGG TGACTTGTCG AGCCCA 516

(2) INFORMATION FOR SEQ ID NO:4:

(i) SEQUENCE CHARACTERISTICS:
(A) LENGTH: 172 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO:4:

Cys Asp Leu Ser Gln Asn His Val Leu Val Gly Arg Lys Asn Leu Arg
1               5                   10                  15

Leu Leu Asp Glu Met Arg Arg Leu Ser Pro His Phe Cys Leu Gln Asp
            20                  25                  30

Arg Lys Asp Phe Ala Leu Pro Gln Glu Met Val Glu Gly Gly Gln Leu
        35                  40                  45

Gln Glu Ala Gln Ala Ile Ser Val Leu His Glu Met Leu Gln Gln Ser
    50                  55                  60

Phe Asn Leu Phe His Thr Glu His Ser Ser Ala Ala Trp Asp Thr Thr
65                  70                  75                  80

Leu Leu Glu Pro Cys Arg Thr Gly Leu His Gln Gln Leu Asp Asn Leu
                85                  90                  95

Asp Ala Cys Leu Gly Gln Val Met Gly Glu Glu Asp Ser Ala Leu Gly
            100                 105                 110

Arg Thr Gly Pro Thr Leu Ala Leu Lys Arg Tyr Phe Gln Gly Ile His
        115                 120                 125

Val Tyr Leu Lys Glu Lys Gly Tyr Ser Asp Cys Ala Trp Glu Thr Val
    130                 135                 140

Arg Leu Glu Ile Met Arg Ser Phe Ser Ser Leu Ile Ser Leu Gln Glu
145                 150                 155                 160

Arg Leu Arg Met Met Asp Gly Asp Leu Ser Ser Pro
                165                 170

(2) INFORMATION FOR SEQ ID NO:5:

(i) SEQUENCE CHARACTERISTICS:
(A) LENGTH: 37 amino acids
(B) TYPE: amino acid
(D) TOPOLOGY: linear

(ii) MOLECULE TYPE: protein

(xi) SEQUENCE DESCRIPTION: SEQ ID NO:5:

Cys Tyr Leu Ser Arg Lys Leu Met Leu Asp Ala Arg Glu Asn Leu Lys
                5                   10                  15

Leu Leu Asp Arg Met Asn Arg Leu Ser Pro His Ser Cys Leu Gln Asp
            20                  25                  30

Arg Lys Asp Phe Gly
        35

(2) INFORMATION FOR SEQ ID NO:6:

(i) SEQUENCE CHARACTERISTICS:
(A) LENGTH: 31 amino acids

(B) TYPE: amino acid
(D) TOPOLOGY: linear

(ii) MOLECULE TYPE: protein

(xi) SEQUENCE DESCRIPTION: SEQ ID NO:6:

Lys Asp Phe Gly Leu Pro Gln Glu Met Val Glu Gly Asp Gln Leu Gln
                5                   10                  15

Lys Asp Gln Ala Phe Pro Val Leu Tyr Glu Met Leu Gln Gln Ser
            20                  25                  30

(2) INFORMATION FOR SEQ ID NO:7:

(i) SEQUENCE CHARACTERISTICS:
(A) LENGTH: 31 amino acids
(B) TYPE: amino acid
(D) TOPOLOGY: linear

(ii) MOLECULE TYPE: protein

(xi) SEQUENCE DESCRIPTION: SEQ ID NO:7:

Gln Gln Ser Phe Asn Leu Phe Tyr Thr Glu His Ser Ser Ala Ala Trp
                5                   10                  15

Asp Thr Thr Leu Leu Glu Gln Leu Cys Thr Gly Leu Gln Gln Gln
            20                  25                  30

(2) INFORMATION FOR SEQ ID NO:8:

(i) SEQUENCE CHARACTERISTICS:
(A) LENGTH: 33 amino acids
(B) TYPE: amino acid
(D) TOPOLOGY: linear

(ii) MOLECULE TYPE: protein

(xi) SEQUENCE DESCRIPTION: SEQ ID NO:8:

Gln Gln Gln Leu Asp His Leu Asp Thr Cys Arg Gly Gln Val Met Gly
                5                   10                  15

Glu Glu Asp Ser Glu Leu Gly Asn Met Asp Pro Ile Val Thr Val Lys
            20                  25                  30

Lys

(2) INFORMATION FOR SEQ ID NO:9:

(i) SEQUENCE CHARACTERISTICS:
(A) LENGTH: 32 amino acids
(B) TYPE: amino acid
(D) TOPOLOGY: linear

(ii) MOLECULE TYPE: protein

(xi) SEQUENCE DESCRIPTION: SEQ ID NO:9:

Thr Val Lys Lys Tyr Phe Gln Gly Ile Tyr Asp Tyr Leu Gln Glu Lys
                5                   10                  15

Gly Tyr Ser Asp Cys Ala Trp Glu Ile Val Arg Val Glu Met Met Arg
            20                  25                  30

(2) INFORMATION FOR SEQ ID NO:10:

(i) SEQUENCE CHARACTERISTICS:
(A) LENGTH: 34 amino acids
(B) TYPE: amino acid
(D) TOPOLOGY: linear

(ii) MOLECULE TYPE: protein

(xi) SEQUENCE DESCRIPTION: SEQ ID NO:10:

Cys Ala Trp Glu Ile Val Arg Val Glu Met Met Arg Ala Leu Thr Val
                5                   10                  15

Ser Thr Thr Leu Gln Lys Arg Leu Thr Lys Met Gly Gly Asp Leu Asn
            20                  25                  30

Ser Pro

(2) INFORMATION FOR SEQ ID NO:11:

    (i) SEQUENCE CHARACTERISTICS:
        (A) LENGTH: 588 base pairs
        (B) TYPE: nucleic acid
        (C) STRANDEDNESS: double
        (D) TOPOLOGY: linear

    (ii) MOLECULE TYPE: DNA (genomic)

    (iii) HYPOTHETICAL: NO

    (iv) ANTI-SENSE: NO

    (vi) ORIGINAL SOURCE:
        (C) INDIVIDUAL ISOLATE: Human Interferon Tau Coding
            Sequences

    (ix) FEATURE:
        (A) NAME/KEY: CDS
        (B) LOCATION: 1..585

    (xi) SEQUENCE DESCRIPTION: SEQ ID NO:11:
ATG GCC TTC GTG CTC TCT CTA CTC ATG GCC CTG GTG CTG GTC AGC TAC      48
GGC CCA GGA GGA TCC CTG GGT TGT GAC CTG TCT CAG AAC CAC GTG CTG      96
GTT GGC AGG AAG AAC CTC AGG CTC CTG GAC GAA ATG AGG AGA CTC TCC 144
CCT CAC TTT TGT CTG CAG GAC AGA AAA GAC TTC GCT TTA CCC CAG GAA 192
ATG GTG GAG GGC GGC CAG CTC CAG GAG GCC CAG GCC ATC TCT GTG CTC 240
CAT GAG ATG CTC CAG CAG AGC TTC AAC CTC TTC CAC ACA GAG CAC TCC 288
TCT GCT GCC TGG GAC ACC ACC CTC CTG GAG CCA TGC CGC ACT GGA CTC 336
CAT CAG CAG CTG GAC AAC CTG GAT GCC TGC CTG GGG CAG GTG ATG GGA 384
GAG GAA GAC TCT GCC CTG GGA AGG ACG GGC CCC ACC CTG GCT CTG AAG 432
AGG TAC TTC CAG GGC ATC CAT GTC TAC CTG AAA GAG AAG GGA TAC AGC 480
GAC TGC GCC TGG GAA ACC GTC AGA CTG GAA ATC ATG AGA TCC TTC TCT 528
TCA TTA ATC AGC TTG CAA GAA AGG TTA AGA ATG ATG GAT GGA GAC CTG 576
AGC TCA CCT TGA

(2) INFORMATION FOR SEQ ID NO:12:

(i) SEQUENCE CHARACTERISTICS:
(A) LENGTH: 195 amino acids
(B) TYPE: amino acid
(D) TOPOLOGY: linear

(ii) MOLECULE TYPE: protein

(xi) SEQUENCE DESCRIPTION: SEQ ID NO:12:

(2) INFORMATION FOR SEQ ID NO:13:

     (i) SEQUENCE CHARACTERISTICS:
          (A) LENGTH: 25 bases
          (B) TYPE: nucleic acid
          (C) STRANDEDNESS: single
          (D) TOPOLOGY: linear

    (ii) MOLECULE TYPE: DNA (synthetic)

    (xi) SEQUENCE DESCRIPTION: SEQ ID NO:13:
CCTGTCTGCA GGACAGAAAA GACTT                                          25

(2) INFORMATION FOR SEQ ID NO:14:

     (i) SEQUENCE CHARACTERISTICS:
          (A) LENGTH: 25 bases
          (B) TYPE: nucleic acid
          (C) STRANDEDNESS: single
          (D) TOPOLOGY: linear

    (ii) MOLECULE TYPE: DNA (synthetic)

    (xi) SEQUENCE DESCRIPTION: SEQ ID NO:14:
TCTGAATTCT GACGATTTCC CAGGC                                          25

(2) INFORMATION FOR SEQ ID NO:15:

(i) SEQUENCE CHARACTERISTICS:
(A) LENGTH: 37 amino acids
(B) TYPE: amino acid
(D) TOPOLOGY: linear

(ii) MOLECULE TYPE: peptide

(iii) HYPOTHETICAL: NO

(vi) ORIGINAL SOURCE:
(C) INDIVIDUAL ISOLATE: Amino acid sequence of fragment
1-37, Human Tau-IFN

(xi) SEQUENCE DESCRIPTION: SEQ ID NO:15:

Cys Asp Leu Ser Gln Asn His Val Leu Val Gly Arg Lys Asn Leu Arg
1               5                   10                  15

Leu Leu Asp Glu Met Arg Arg Leu Ser Pro His Phe Cys Leu Gln Asp
            20                  25                  30

Arg Lys Asp Phe Ala
        35

(2) INFORMATION FOR SEQ ID NO:16:

(i) SEQUENCE CHARACTERISTICS:
(A) LENGTH: 31 amino acids
(B) TYPE: amino acid
(D) TOPOLOGY: linear

(ii) MOLECULE TYPE: peptide

(iii) HYPOTHETICAL: NO

(vi) ORIGINAL SOURCE:
(C) INDIVIDUAL ISOLATE: Amino acid sequence of fragment

34-64, Human Tau-IFN

(xi) SEQUENCE DESCRIPTION: SEQ ID NO:16:

Lys Asp Phe Ala Leu Pro Gln Glu Met Val Glu Gly Gly Gln Leu Gln
1               5                   10                  15

Glu Ala Gln Ala Ile Ser Val Leu His Glu Met Leu Gln Gln Ser
            20                  25                  30

(2) INFORMATION FOR SEQ ID NO:17:

(i) SEQUENCE CHARACTERISTICS:
(A) LENGTH: 31 amino acids
(B) TYPE: amino acid
(D) TOPOLOGY: linear

(ii) MOLECULE TYPE: peptide

(iii) HYPOTHETICAL: NO

(vi) ORIGINAL SOURCE:
(C) INDIVIDUAL ISOLATE: Amino acid sequence of fragment
62-92, Human Tau-IFN

(xi) SEQUENCE DESCRIPTION: SEQ ID NO:17:

Gln Gln Ser Phe Asn Leu Phe His Thr Glu His Ser Ser Ala Ala Trp
1               5                   10                  15

Asp Thr Thr Leu Leu Glu Pro Cys Arg Thr Gly Leu His Gln Gln
            20                  25                  30

(2) INFORMATION FOR SEQ ID NO:18:

(i) SEQUENCE CHARACTERISTICS:
(A) LENGTH: 33 amino acids
(B) TYPE: amino acid
(D) TOPOLOGY: linear

(ii) MOLECULE TYPE: peptide

(iii) HYPOTHETICAL: NO

(vi) ORIGINAL SOURCE:
(C) INDIVIDUAL ISOLATE: Amino acid sequence of fragment
90-122, Human Tau-IFN

(xi) SEQUENCE DESCRIPTION: SEQ ID NO:18:

His Gln Gln Leu Asp Asn Leu Asp Ala Cys Leu Gly Gln Val Met Gly
1               5                   10                  15

Glu Glu Asp Ser Ala Leu Gly Arg Thr Gly Pro Thr Leu Ala Leu Lys
            20                  25                  30

Arg

(2) INFORMATION FOR SEQ ID NO:19:

(i) SEQUENCE CHARACTERISTICS:
(A) LENGTH: 32 amino acids
(B) TYPE: amino acid
(D) TOPOLOGY: linear

(ii) MOLECULE TYPE: peptide

(iii) HYPOTHETICAL: NO

(vi) ORIGINAL SOURCE:
(C) INDIVIDUAL ISOLATE: Amino acid sequence of fragment
119-150, Human Tau-IFN

(xi) SEQUENCE DESCRIPTION: SEQ ID NO:19:

Ala Leu Lys Arg Tyr Phe Gln Gly Ile His Val Tyr Leu Lys Glu Lys
1               5                   10                  15

Gly Tyr Ser Asp Cys Ala Trp Glu Thr Val Arg Leu Glu Ile Met Arg
            20                  25                  30

(2) INFORMATION FOR SEQ ID NO:20:

(i) SEQUENCE CHARACTERISTICS:
(A) LENGTH: 34 amino acids
(B) TYPE: amino acid
(D) TOPOLOGY: linear

(ii) MOLECULE TYPE: peptide

(iii) HYPOTHETICAL: NO

(vi) ORIGINAL SOURCE:
(C) INDIVIDUAL ISOLATE: Amino acid sequence of fragment
139-172, Human Tau-IFN

(xi) SEQUENCE DESCRIPTION: SEQ ID NO:20:

Cys Ala Trp Glu Thr Val Arg Leu Glu Ile Met Arg Ser Phe Ser Ser
1               5                   10                  15

Leu Ile Ser Leu Gln Glu Arg Leu Arg Met Met Asp Gly Asp Leu Ser
            20                  25                  30

Ser Pro

## Description

### Field of the Invention

The present invention relates to interferon-τ compositions and methods of use.

### References

Ausubel, F.M., et al., Current Protocols in Molecular Biology, John Wiley and Sons, Inc., Media, PA.
Bazer, F.W., *et al., J. Animal Sci*. 57(Supp. 2):425 (1983).
Bazer, F.W., *et al., J. Reproduction and Fertility* 76:841 (1986).
Bazer, F.W., *et al.,* Biology of Reproduction, vol.40;(Supplement 1):63, (Abstract) (1989).
Beames, *et al.,* Biotechniques 11:378 (1991).
Benoit, P., *et al., J. Immunol*. 150(3):707 (1993).
Bonnem, E.M., *et al., J. Bio. Response Modifiers* 3:580 (1984).
Boyer, S.J., *et al*., J. Biol. Regul. Homeost. Agents. 6(3):99-102 (1992).
Chan, *et al., DNA* 4:165 (1985).
Crea, R., U. S. Patent No. 4,888,286, issued December 19, 1989.
Crowe, S. M., et al., AIDS Res. Hum. Retroviruses 3(2):135 (1987).
Cumber, J. A., et. al., Methods in Enzymology, 112:207 (1985).
Davis, G.L., *et al., N. England J. Med.* 321:1501 (1989).
Davis, G.L., *et al., Theriogenology* 38:867 (1992).
DeMaeyer, E., *et al., Interferons and Other Regulatory Cytokines*, John Wiley and Sons, New York (1988).
Dianzani, *F., J. Interferon Res., Special Issue,* 5/92:109 (1992).
Duncan, R. J. S., et. al., Anal Biochem, 182:68 (1983).
Dusheiko, G.M., *et al., J. Hematology* 3(Supl. 2):S199 (1986).
Eaton, M. A. W., et al., U. S. Patent No. 4,719,180, issued Jan. 12, 1988.
Ecker, D.J., *et al*., J. Biol. Chem. 264:7715-7719 (1989).
Familetti, P.C., *et al., Meth. Enzymol*. 78:387 (1981).
Finter, N.B., *et al., Drugs* 42(5):749 (1991).
Foa, P., *et al.,* Cell Tissue Kinet. 15(4):399-404 (1982).
Francis, M.L., *et al., AIDS Res. and Human Retroviruses* 8(2):199 (1992).
Frangioni, J.V., *et al*., Anal. Biochem. 210(1):179-187 (1993).
Godkin, J.D., *et al., J. Reprod. Fert.* 65:141 (1982).
Griggs, N.D., *et al., J. Immunol*. 149:517 (1992).
Guan, K.L., et *al*., Anal. Biochem. 192(2):262-267 (1991).
Hakes, D.J., *et al*., Anal. Biochem. 202(2):293-298 (1992).
Hansen, P.J., *et al*., U. S. Patent No. 4,997,646, issued 5 March 1991.
Harlow, E., et al., Antibodies: A Laboratory Manual, Cold Spring Harbor Press, Cold Spring Harbor, NY (1988).
Helmer, S.D., *et al*., J. Reprod. Fert. 79:83-91 (1987).
Hitzeman, R.A., *et al*., US Patent No. 4,775,622, issued Oct. 4, 1988.
Hoffman, A.E., et al., Virology 147:326 (1985).
Howatson, *et al*., *J. Endocrinol.* 119:531 (1988).
Imakawa, K., *et al., Nature* 330:377 (1987).
Imakawa, K., *et al*., Mol. Endocrinol. 3:127-139 (1989).
Innis, M., U.S. Patent No. 4,975,276, issued Dec. 4, 1990.
Johnson, W.C., Jr., Methods in Enzymology, Vol. 210, pp. 426-447, (1992).
Kashima, H., *et al., Laryngoscope* 98:334 (1988).
Krown, S.E., in "Mechanisms of Interferon Actions", (Pfeffer, L.M., ed.), CRC Press Inc., Boca Raton, Vol. II, pp. 143-178, (1987).
Kyte, J., *et al., J. Mol. Biol*. 157:105 (1982).
Langford, M.P., *et al., Meth. Enzymol.* 78:339 (1981).
Lawrence, *et al., Nucl. Acids. Res.* 13:1777 (1985).
Lowry, O.H., *et al., J. Biol. Chem.* 193265-275 (1951).
Maniatis, T., et al. Molecular Cloning: A Laboratory Manual, Cold Spring Harbor Laboratory (1982).
Martin, E.W., "In: Dispensing of medication: a practical manual on the formulation and dispensing of pharmaceutical products", (Hoover, J.E. ed.), 8th edition, Mack Publishing Co., Easton, PA., (1976).
Meyer, F., *et al*., U.S. Patent No. 4,885,166, issued Dec. 5, 1989.
McInnes, B., *et al*., J. Interferon Res. 9(3), pp. 305-314, (1989).
Mullis, K.B., U. S. Patent No. 4,683,202, issued 28 July 1987.
Mullis, K.B., et al., U. S. Patent No. 4,683,195, issued 28 July 1987.
Miyoshi, E., *et al.,* Int. J. Cancer 52(1):137-140 (1992).
Oeda, K., *et al*., U.S. Patent No. 4,766,068, issued Aug. 23, 1988.
Oldham, R.K., *Hospital Practice* 20:71 (1985).
Paulesu, *et al., J. Biol. Regul. Homeost. Agents* 5:81 (1991).
Pederson, *et al., Science* 235:790 (1987).
Perczel, A., *et al.,* Protein Engineering, Vol. 4, (Supp. 6), pp. 669-679, (1991).
Pontzer, *et al., Biochem. Biophys. Res. Comm*. 152:801 (1988).
Pontzer, C.H., *et al., Cancer Res.* 51:5304 (1991).
Poste, G., *et al.,* Proc. Nat'l Acad. Sci., USA, Vol. 78:6226 (1981).
Quesada, J.R., *et al., N. England J. Med*. 310:15 (1984).
Reilly, P.R., *et al.,* Baculovirus Expression Vectors: A Laboratory Manual (1992).
Roberts, R.M., *et al., Endocrine Reviews* 13:432 (1992).
Rothstein, R., DNA Cloning: A Practical Approach, vol. II (Glover, D.M., Ed.) Oxford: IRL Press, pp. 46-66 (1986).
Ruegg, C.L., et al., *J. Interferon Res.,* 10:621-626, (1990).
Rutter, W.J., *et al*., *U.S.* Patent No. 4,769,238, issued Sept. 6, 1988.
Sabin, E., *et al.,* Bio/Technology 7:705-709 (1989).
Sambrook, J., *et al.,* Molecular Cloning, A Laboratory Manual (Cold Spring Harbor Laboratory, Cold Spring Harbor, NY) (1989).
Sanger, *et al., Proc. Natl. Acad. Sci. USA* 74:5463 (1977).
Senda, T., *et al.,* EMBO J., Vol. 11, Supp. 9, pp. 3193-3201, (1992).
Shoulders, C.C., *et al.,* Nucleic Acids Res. 10(16):4873-4882 (1982).
Smith, D.B., *et al.,* Gene 67:31 (1988).
Smith, P.K., *et al., Anal. Biochem*. 150:76 (1985).
Stewart, H.J., *et al., J. Endocrinol.* 115:R13 (1987).
Todd, R.F., *et al.,* Leuk. Res. 5(6):491-495 (1981).
Vallet, J.L., *et al., Biol. Reprod.* 37:1307 (1987).
Vallet, J.L., *et al., J. Endocrinology* 117:R5-R8 (1988).
Wallis, S.C., *et al.,* EMBO J. 2(12):2369-2373 (1983).
Wang, C. Y., et al., U.S. Patent No. 4,879,212, issued 7 Nov. 1989.
Wang, C. Y., et al., U.S. Patent No. 4,735,896, issue 5 April 1988.
Wilson, *et al., Biology of Reproduction* 20(Supp. 1):101A, Abstract (1979).
Yoshio, T., et al., U. S. Patent No. 4,849,350, issued July 18, 1989.
Young, *et al., Proc. Natl. Acad. Sci. USA* 80:1194 (1983).

### Background of the Invention

Conceptus membranes, or trophectoderm, of various mammals produce biochemical signals that allow for the establishment and maintenance of pregnancy (Bazer, *et al.,* 1983). One such protein, ovine trophoblast protein-one (oTP-1), was identified as a low molecular weight protein secreted by sheep conceptuses between days 10 and 21 of pregnancy (Wilson, *et al.,* 1979; Bazer, *et al.,* 1986). The protein oTP-1 was shown to inhibit uterine secretion of prostaglandin F₂-alpha, which causes the corpus luteum on the ovary to undergo physiological and endocrinological demise in nonpregnant sheep (Bazer, et *al.,* 1986). Accordingly, oTP-1 has antiluteolytic biological activity. The primary role of oTP-1 was assumed to be associated with the establishment of pregnancy.

oTP-1 was subsequently found to (i) exhibit limited homology (50-70%) with interferon alphas (IFNα) of various species (Imakawa, *et al.,* 1987), and (ii) bind to a Type I interferon receptor (Stewart, *et al*., 1987). Despite some similarities with IFNα, oTP-1 has several features that distinguish it from IFNα including the following: oTP-1's role in reproductive biochemistry (other interferons are not known to have any role in the biochemical regulation of reproductive cycles), oTP-1's cellular source -- trophoblast cells (IFNα is derived from lymphocytes cells), oTP-1's size -- 172 amino acids (IFNα is typically about 166 amino acids), and oTP-1 is weakly inducible by viruses (IFNα is highly inducible by viruses). The International Interferon Society recognizes oTP-1 as belonging to an entirely new class of interferons which have been named interferon-tau (IFNτ). The Greek letter τ stands for trophoblast.

The interferons have been classified into two distinct groups: type I interferons, including IFNα, IFNβ, and IFNω (also known as IFNαII); and type II interferons, represented by IFNγ (reviewed by DeMaeyer, *et al*.). In humans, it is estimated that there are at least 17 IFNα non-allelic genes, at least about 2 or 3 IFNβ non-allelic genes, and a single IFNγ gene.

IFNα's has been shown to inhibit various types of cellular proliferation. IFNα's are especially useful against hematologic malignancies such as hairy-cell leukemia (Quesada, *et al.,* 1984). Further, these proteins have also shown activity against multiple myeloma, chronic lymphocytic leukemia, low-grade lymphoma, Kaposi's sarcoma, chronic myelogenous leukemia, renal-cell carcinoma, urinary bladder tumors and ovarian cancers (Bonnem, et al., 1984; Oldham, 1985). The role of interferons and interferon receptors in the pathogenesis of certain autoimmune and inflammatory diseases has also been investigated (Benoit, *et al.,* 1993).

IFNα's are also useful against various types of viral infections (Finter, *et al.,* 1991). Alpha interferons have shown activity against human papillomavirus infection, Hepatitis B, and Hepatitis C infections (Finter, *et al.,* 1991; Kashima, *et al.,* 1988; Dusheiko, *et al.,* 1986; Davis, *et al.,* 1989). Significantly, however, the usefulness of IFNα's has been limited by their toxicity: use of interferons in the treatment of cancer and viral disease has resulted in serious side effects, such as fever, chills, anorexia, weight loss, and fatigue (Pontzer, *et al.,* 1991; Oldham, 1985). These side effects often require (i) the interferon dosage to be reduced to levels that limit the effectiveness of treatment, or (ii) the removal of the patient from treatment. Such toxicity has reduced the usefulness of these potent antiviral and antiproliferative proteins in the treatment of debilitating human and animal diseases.

### Summary of the Invention

In one aspect the present invention includes a method of inhibiting tumor cell growth. In the method, cells are contacted with interferon-τ (IFNτ) at a concentration effective to inhibit growth of the tumor cells. IFNτ can be obtained from a number of sources including cows, sheep, and humans. Two embodiments include the IFNτ presented as either SEQ ID NO:2 or SEQ ID NO:4. A number-of tumor cells can be targeted for growth inhibition by IFNτ, including but not limited to the following: human carcinoma cells and steroid-affected tumor cells (*e.g*., mammary tumor cells).

The present invention also includes a method of inhibiting viral replication in a cell. In this method cells infected with a virus are contacted with interferon-τ at a concentration effective to inhibit viral replication within the cells. The IFNτ molecules described above are also useful in this method of the present invention. The replication of a number of viruses can be inhibited in cells, these viruses include RNA (*e.g*., feline leukemia virus, human immunodeficiency virus, or Hepatitis C Virus) and DNA (e.g., Hepatitis B Virus) viruses.

The human IFNτ molecules of the present invention can also be used in a method of enhancing fertility in a female mammal. In this method an amount of human IFNτ effective to enhance fertility of a female mammal is administered, typically in a pharmaceutically acceptable carrier. Exemplary of such human IFNτ molecules are the protein sequences presented as SEQ ID NO:4 and SEQ ID NO:12.

The present invention also includes an isolated nucleic acid which encodes a human interferon-τ. Exemplary of such nucleic acid molecules are SEQ ID NO:3 and SEQ ID NO:11. Further, the invention includes expression vectors for the expression of human IFNτ. Typically the expression vector includes (a) a nucleic acid containing an open reading frame that encodes human interferon-τ; and (b) regulatory sequences effective to express said open reading frame in a host cell. The regulatory sequence may include sequences useful for targeting or secretion of the IFNτ polypeptide: such sequences may be endogenous (such as the normally occurring IFNτ leader sequences, see SEQ ID NO:11) or heterologous (such as a secretory signal recognized in yeast or bacterial expression systems). In the expression vector, regulatory sequences may also include, 5' to said nucleic acid sequence, a promoter region and an ATG start codon in-frame with the interferon-τ coding sequence, and 3' to said coding sequence, a translation termination signal followed by a transcription termination signal. The nucleic acid in the expression vector may be selected from, for example, SEQ ID NO:1, SEQ ID NO:3 and SEQ ID NO:11.

In another embodiment, the present invention includes a recombinantly produced human interferon-τ protein. One exemplary sequence for such a protein is given as SEQ ID NO:4. Human IFNτ may also contain a carboxy terminal extension (such as the sequence presented as SEQ ID NO:12).

The invention includes a method of recombinantly producing interferon-τ. In the method, a recombinant expression system containing an open reading frame (ORF) having a polynucleotide sequence which encodes a human interferon-τ polypeptide, where the vector is designed to express the ORF in the host, is introduced into suitable host cells. The host is then cultured under conditions resulting in the expression of the ORF sequence. The human IFNτ sequences discussed above are examples of suitable human IFNτ polypeptides. Examples of polynucleotide coding sequences are SEQ ID NO:3 and SEQ ID NO:11. Numerous vectors and their corresponding hosts are useful in the practice of this method of the invention, including, lambda gtl1 phage vector and *E. coli* cells. Other host cells include, yeast and insect cell expression systems.

The invention further includes expression systems useful for the expression of interferon-τ polypeptides. Typically these systems include a host capable of supporting expression of an open reading frame in a selected expression vector, and the selected expression vector containing an open reading frame (ORF) having a polynucleotide sequence which encodes a human interferon-τ polypeptide. Exemplary of sequences that can be utilized in such expression systems are SEQ ID NO:4, SEQ ID NO:15, SEQ ID NO:16, SEQ ID NO:17, SEQ ID NO:18, SEQ ID NO:19 and SEQ ID NO:20.

The invention also includes isolated interferon-τ polypeptides. These polypeptides are derived from the interferon-τ amino acid coding sequence and are between about 15 and 172 amino acids in length. Such polypeptides can be selected, for example, from the sequences presented as SEQ ID NO:2 and SEQ ID NO:4. Exemplary IFNτ-derived polypeptides include, but are not limited to, the following: SEQ ID NO:5, SEQ ID NO:7, SEQ ID NO:10, SEQ ID NO:15, SEQ ID NO:17, and SEQ ID NO:20.

In another embodiment, the invention includes a method of blocking the binding of alpha-interferon to a cell having an alpha-interferon receptor. In this method the cell is contacted with an interferon-τ polypeptide at a concentration effective to allow the binding of interferon-τ to each alpha-interferon receptor. The cells, having the IFNτ polypeptide bound to the receptor, are then exposed to alpha-interferon (IFNα). The IFNτ polypeptides and IFNτ-derived polypeptides described above are examples of IFNτ polypeptides useful in this method.

Further, the invention includes a method of blocking the binding of interferon-τ to a cell having a interferon-τ receptor. In this method, the cell is contacted with an interferon-τ-derived polypeptide (*e.g.*, SEQ ID NO:5, SEQ ID NO:7, SEQ ID NO:10, SEQ ID NO:15, SEQ ID NO:17 or SEQ ID NO:20), where the polypeptide is at a concentration effective to allow the binding of the polypeptide to each interferon-τ-receptor. The cells are then exposed to interferon-τ.

The invention also includes purified antibodies that are immunoreactive with human interferon-τ. The antibodies may be polyclonal or monoclonal. Exemplary IFNτ polypeptide antigens include, but are not limited, to the following: SEQ ID NO:4, SEQ ID NO:15, SEQ ID NO:17 and SEQ ID NO:20.

The present invention also includes the following: interferon-τ-derived polypeptides that have anti-tumor (*i.e*., anti-proliferative) activity; interferon-τ-derived polypeptides that have anti-viral activity; and hybrid α-interferon molecules in which the toxicity portion of native IFNα has been replaced by analogous sequences from IFNτ.

These and other objects and features of the invention will be more fully appreciated when the following detailed description of the invention is read in conjunction with the accompanying drawings.

### Brief Description of the Figures

Figure 1 presents the nucleic acid coding sequence of a synthetic gene of OvIFNτ designed to include 19 unique restriction enzyme sites spaced evenly throughout the coding sequence.
Figure 2 shows the cloning strategy used for making a synthetic gene encoding OvIFNτ.
Figure 3 shows a comparison of the predicted protein sequences of a human interferon-τ gene and an ovine interferon-τ gene. Divergent amino acids are indicated by presentation of the alternative amino acid on the line below the nucleic acid sequences.
Figure 4 presents data demonstrating that both OvIFNτ and IFNα were able to drastically reduce growth of HL-60 cells.
Figure 5 presents data demonstrating that rHuIFNα is cytotoxic and OvIFNτ is not. In the figure, results of one of three replicate experiments are presented as mean % viability ± SD.
Figure 6 presents the sequences of polypeptides derived from the IFNτ sequence.
Figure 7 presents the complete nucleic acid and amino acid sequence of an OvIFNτ sequence.
Figure 8 presents data supporting the lack of cytotoxicity, relative to IFNα, when IFNτ is used to treat peripheral blood mononuclear cells.
Figure 9 shows the results of treatment of a human cutaneous T cell lymphoma line, HUT 78, with IFNτ.
Figure 10 shows the results of treatment of a human T cell lymphoma line, H9, with IFNτ.
Figure 11A presents data for the peptide inhibition, relative to FIV (feline immunodeficiency virus) replication, of polypeptides derived from OvIFNτ with whole OvIFNτ. Figure 11B presents data for the peptide inhibition, relative to HIV (human immunodeficiency virus) replication, of polypeptides derived from OvIFNτ with whole OvIFNτ.
Figure 12 presents data demonstrating the inhibition of the antiviral activity of IFNτ by IFNτ-derived peptides.
Figure 13 presents data demonstrating the inhibition by IFNτ-derived peptides of OvIFNτ antiviral activity.
Figure 14 presents data demonstrating the inhibition by IFNτ-derived peptides of bovine IFNα antiviral activity.
Figure 15 presents data demonstrating the inhibition by IFNτ-derived peptides of human IFNα antiviral activity.
Figure 16 presents data evaluating the lack of inhibition by IFNτ-derived peptides of bovine IFNγ anti-viral activity.
Figure 17 presents data demonstrating the anti-IFNτ-derived peptide antisera inhibition of the antiviral activity of IFNτ.
Figure 18 presents data demonstrating the anti-IFNτ-derived peptide antisera inhibition of the binding of radiolabeled IFNτ to cells.

### Brief Description of the Sequences

SEQ ID NO:1 is the nucleotide sequence of a synthetic gene encoding ovine interferon-τ (OvIFNτ). Also shown is the encoded amino acid sequence.

SEQ ID NO:2 is an amino acid sequence of a mature OvIFNτ protein.

SEQ ID NO:3 is a synthetic nucleotide sequence encoding a mature human interferon-τ (HuIFNτ) protein.

SEQ ID NO:4 is an amino acid sequence for a mature HuIFNτ protein.

SEQ ID NO:5 is the amino acid sequence of fragment 1-37 of SEQ ID NO:2.

SEQ ID NO:6 is the amino acid sequence of fragment 34-64 of SEQ ID NO:2.

SEQ ID NO:7 is the amino acid sequence of fragment 62-92 of SEQ ID NO:2.

SEQ ID NO:8 is the amino acid sequence of fragment 90-122 of SEQ ID NO:2.

SEQ ID NO:9 is the amino acid sequence of fragment 119-150 of SEQ ID NO:2.

SEQ ID NO:10 is the amino acid sequence of fragment 139-172 of SEQ ID NO:2.

SEQ ID NO:11 is the nucleotide sequence of a natural HuIFNτ gene with a leader sequence.

SEQ ID NO:12 is the predicted amino acid coding sequence of the SEQ ID NO:11.

SEQ ID NO:13 is a 25-mer synthetic oligonucleotide according to the subject invention.

SEQ ID NO:14 is a 25-mer synthetic oligonucleotide according the subject invention.

SEQ ID NO:15 is the amino acid sequence of fragment 1-37 of SEQ ID NO:4.

SEQ ID NO:16 is the amino acid sequence of fragment 34-64 of SEQ ID NO:4.

SEQ ID NO:17 is the amino acid sequence of fragment 62-92 of SEQ ID NO:4.

SEQ ID NO:18 is the amino acid sequence of fragment 90-122 of SEQ ID NO:4.

SEQ ID NO:19 is the amino acid sequence of fragment 119-150 of SEQ ID NO:4.

SEQ ID NO:20 is the amino acid sequence of fragment 139-172 of SEQ ID NO:4.

### Detailed Description of the Invention

### I. Definitions.

*Interferon-*τ refers to any one of a family of interferon proteins having the following characteristics: (i) anti-luteolytic properties; (ii) anti-viral properties; (iii) anticellular proliferation properties; (iv) 45-68% amino acid homology with α-Interferons and greater than 70% amino acid homology to the sequence presented as SEQ ID NO:2. Interferon-τ can be isolated from a number of mammalian sources as described below.

An *interferon-τ polypeptide* is a polypeptide having between about 15 and 172 amino acids derived from an interferon-τ amino acid coding sequence, where said 15 to 172 amino acids are contiguous in native interferon-τ. Such 15-172 amino acid regions can also be assembled into polypeptides where two or more such interferon-τ regions are joined that are normally discontinuous in the native protein.

### II. Isolation and Characterization of Interferon-τ.

### A. Ovine and Bovine Interferon-τ.

### 1. Interferon-τ Coding Sequences.

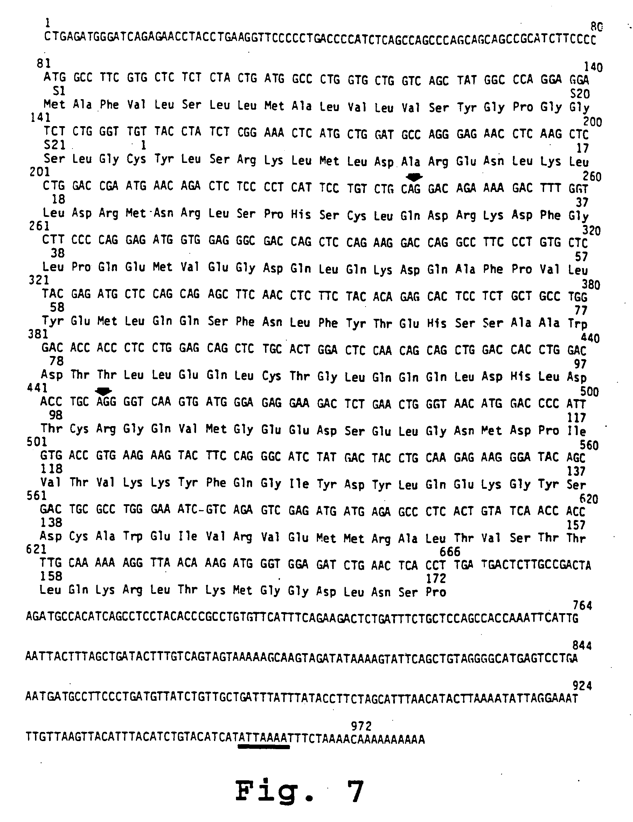

Ovine interferon-τ (OvIFNτ) is a major conceptus secretory protein produced by the embryonic trophectoderm during the critical period of maternal recognition in sheep. One isolate of mature OvIFNτ is 172 amino acids in length (SEQ ID NO:2). The cDNA coding sequence contains an additional 23 amino acids at the amino-terminal end of the mature protein (Imakawa, *et al.,* 1987). The coding sequence of this OvIFNτ isolate is presented as Figure 7.

For the isolation of OvIFNτ protein, conceptuses were collected from pregnant sheep and cultured *in vitro* in a modified Minimum Essential Medium as described previously (Godkin, *et al.,* 1982). Conceptuses were collected on various days of pregnancy with the first day of mating being described as Day 0. IFNτ was purified from conceptus culture medium essentially as described by Vallet, *et al.,* (1987) and Godkin, *et al.* (1982).

The homogeneity of IFNτ was assessed by sodium dodecyl sulfate polyacrylamide gel electrophoresis (SDS-PAGE; Maniatis, *et al.;* Ausubel, *et al*.). Determination of protein concentration in purified IFNτ samples was performed using the bicinchoninic (BCA) assay (Pierce Chemical Co., Rockford, IL; Smith, *et al.,* 1985).

A homologous protein to OvIFNτ was isolated from cows (bIFNτ; Helmer, *et al.,* 1987; Imakawa, *et al.,* 1989). OvIFNτ and BoIFNτ (i) have similar functions in maternal recognition of pregnancy, and (ii) share a high degree of amino acid and nucleotide sequence homology between mature proteins. The nucleic acid sequence homology between OvIFNτ and BoIFNτ is 76.3% for the 5' non-coding region, 89.7% for the coding region, and 91.9% for the 3' non-coding region. The amino acid sequence homology is 80.4%.

Example 1 describes the reproductive functions of OvIFNτ. OvIFNτ and recombinant human α-2-Interferon (rHuIFNα₂) were infused into uterine lumen of ewes at a variety of concentrations. The life span of the corpus luteum was assessed by examination of interestrous intervals, maintenance of progesterone secretion, and inhibition of prostaglandin secretion (Davis, *et al*., *1992*). Comparison of the data resulting from these examinations demonstrated a considerable lengthening of the interestrous interval when IFNτ is administered at 100µg/day and no meaningful effect when rHuIFNα is administered. These data support the conclusion that IFNτ significantly influences the biochemical events of the estrous cycle.

The antiviral properties of interferon-τ at various stages of the reproductive cycle were also examined (Example 2). Conceptus cultures were established using conceptus obtained from sheep at days 12 through 16 of the estrus cycle. Antiviral activity of supernatant from each conceptus culture was assessed. Culture supernatants had increasing antiviral activity associated with advancing development of the conceptus up to Day 16 post estrus.

### 2. Recombinant Production of IFNτ.

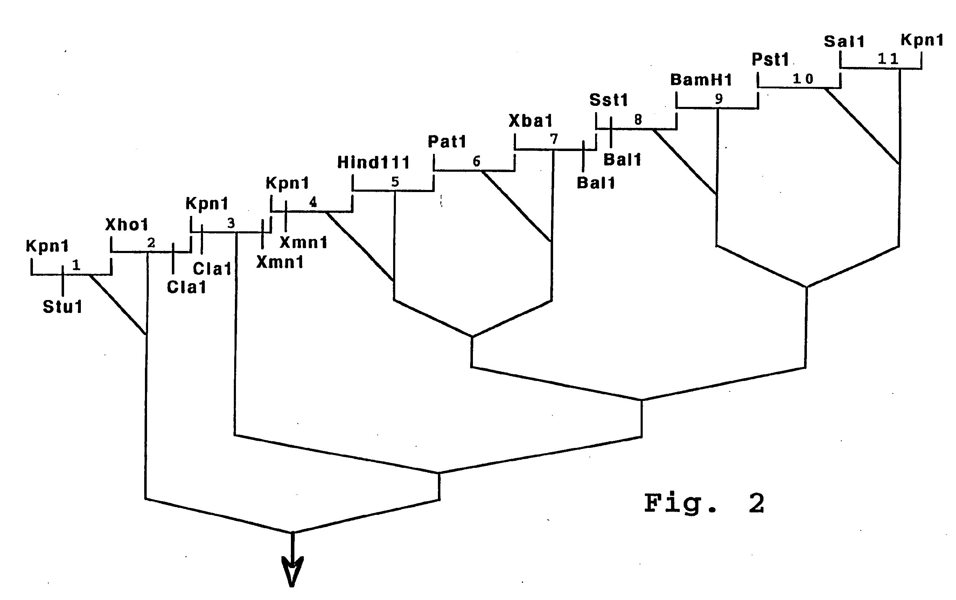

Recombinant IFNτ was produced using bacterial and yeast cells. The amino acid coding sequence for OvIFNτ was used to generate a corresponding DNA coding sequence with codon usage optimized for expression in *E. coli* (Example 3). The DNA coding sequence was synthetically constructed by sequential addition of oligonucleotides. Cloned oligonucleotides were fused into a single polynucleotide using the restriction digestions and ligations outlined in Figure 2. The polynucleotide coding sequence had the sequence presented as SEQ ID NO:1.

For expression of recombinant IFNτ, this synthetic coding sequence can be placed in a number of bacterial expression vectors: for example, lambda gtl1 (Promega, Madison WI); pGEX (Smith, *et al.);* pNH vectors (Stratagene, La Jolla CA). Cloning of the IFNτ synthetic polynucleotide into a modified pIN III omp-A expression vector is described in Example 3. Production of the IFNτ protein was induced by the addition of IPTG. Soluble recombinant IFNτ was liberated from the cells by sonication or osmotic fractionation.

The protein can be further purified by standard methods, including size fractionation (column chromatography or preoperative gel electrophoresis) or affinity chromatography (using, for example, anti-IFNτ antibodies (solid support available from Pharmacia, Piscataway NJ). Protein preparations can also be concentrated by, for example, filtration (Amicon, Danvers, Mass.).

The synthetic IFNτ gene was also cloned into the yeast cloning vector pBS24Ub (Example 4; Sabin, *et al.;* Ecker, *et al.).* Synthetic linkers were constructed to permit in-frame fusion of the IFNτ coding sequences with the ubiquitin coding sequences in the vector. The resulting junction allowed *in vivo* cleavage of the ubiquitin sequences from the IFNτ sequences.

The recombinant plasmid pBS24Ub-IFNτ was transformed into the yeast *S. cerevisiae.* Transformed yeast cells were cultured, lysed and the recombinant IFNτ (r-IFNτ) protein isolated from the cell lysates.

The amount of r-IFNτ was quantified by radioimmunoassay. Microsequencing of the purified r-IFNτ was carried out. The results demonstrated identity with native IFNτ through the first 15 amino acids. The results also confirmed that the ubiquitin/IFNτ fusion protein was correctly processed *in vivo.*

Recombinant IFNτ obtained by this method exhibited antiviral activity similar to the antiviral activity of IFNτ purified from conceptus-conditioned culture medium.

Other yeast vectors can be used in the practice of the present invention including, but are not limited to, vectors with regulatable expression (Hitzeman, *et al.;* Rutter, *et al.;* Oeda, *et al*.). The yeast transformation host is typically *Saccharomyces cerevisiae,* however, other yeast suitable for transformation can be used as well (*e.g., Schizosaccharomyces pombe*).

The DNA encoding the IFNτ polypeptide can be cloned into any number of commercially available vectors to generate expression of the polypeptide in the appropriate host system. These systems include the above described bacterial and yeast expression systems as well as the following: baculovirus expression (Reilly, *et al*.; Beames, *et al*.; Clontech, Palo Alto CA); and expression in mammalian cells (Clontech, Palo Alto CA; Gibco-BRL, Gaithersburg MD). These recombinant polypeptides can be expressed as fusion proteins or as native proteins. A number of features can be engineered into the expression vectors, such as leader sequences which promote the secretion of the expressed sequences into culture medium. The recombinantly produced polypeptides are typically isolated from lysed cells or culture media. Purification can be carried out by methods known in the art including salt fractionation, ion exchange chromatography, and affinity chromatography. Immunoaffinity chromatography can be employed, as described above, using antibodies generated based on the IFNτ polypeptides.

### B. Human Interferon-τ.

### 1. Identification and Cloning of Human Genomic Sequences Encoding an Interferon-τ Protein.

DNA was screened for sequences homologous to interferon-τ (Example 5). Several sequences that hybridized with the OvIFNτ cDNA probe were identified. Several clones containing partial sequences of human interferon-τ were then isolated (Example 6). Two synthetic 25-mer oligonucleotides, corresponding to sequences from the OvIFNτ cDNA (Imakawa, *et al.,* 1987) were synthesized. These primers were employed in amplification reactions using DNA derived from the following two cDNA libraries: human term placenta and human term cytotrophoblast. The resulting amplified DNA fragments were electrophoretically separated and a band containing an IFNτ amplification product was isolated. The product was subcloned and the inserted amplification product sequenced using the dideoxy termination method.

Comparison of sequences from three of the clones revealed a high degree of sequence homology between the isolates, but the sequences were not identical. This result suggests the existence of multiple variants of human interferon-τ genes.

Example 7 describes the isolation of a full-length human IFNτ gene. High molecular weight DNA was isolated from peripheral blood mononuclear cells (PBMCs) and size-fractionated. Fractions were tested for the presence of IFNτ sequences using polymerase chain reaction: DNA molecules from fractions that tested amplification positive were used to generate a subgenomic library in λgt11.

This subgenomic library was plated and hybridized with an OvIFNτ cDNA probe (Example 7A). Approximately 20 clones were identified that hybridized to the probe. Plaques corresponding to the positive clones were passaged, DNA isolated and analyzed by amplification reactions using OvIFNτ primers. Of these twenty plaques, six plaques generated positive PCR signals. The phage from these six clones were purified and the inserts sequenced. One of the inserts from one of these six clones was used as a hybridization probe in the following screening.

Recombinant phage from the λgt11 subgenomic library were screened using the hybridization probe just described (Example 7B). Three clones giving positive hybridization signals were isolated and the inserts sequenced. The resulting nucleic acid sequence information is presented as SEQ ID NO:11 and the predicted protein coding sequence is presented as SEQ ID NO:12. The predicted mature protein coding sequence is presented as SEQ ID NO:4.

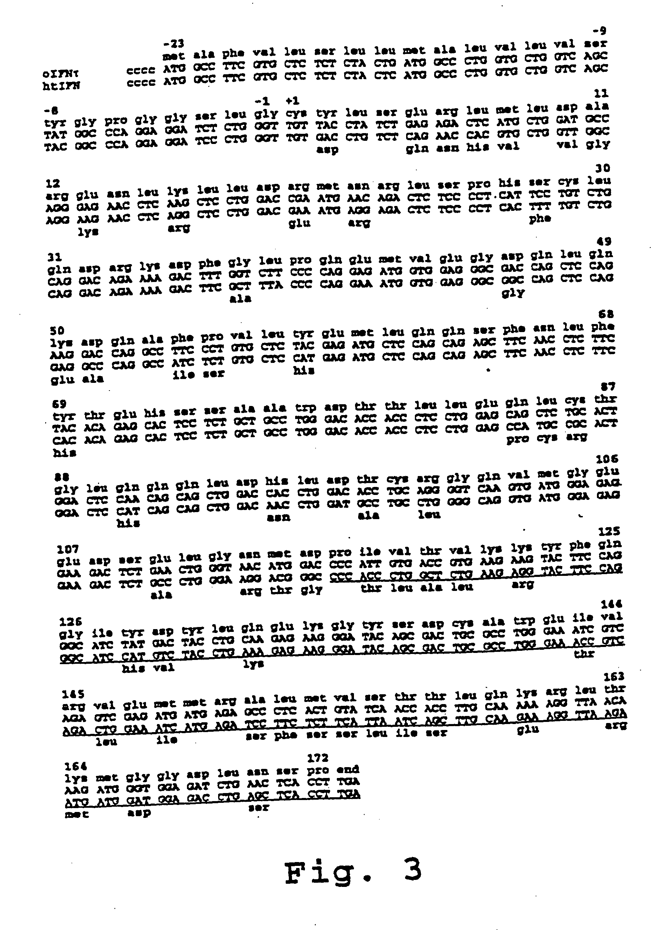

Comparison of the predicted protein sequences (Figure 3) of the human interferon-τ gene (SEQ ID NO:4) and the ovine interferon-τ gene demonstrates the levels of sequence homology and divergence at the amino acid level.

The human IFNτ sequences presented as SEQ ID NO:12 and SEQ ID NO:11, and primers and probes derived therefrom, can be used as specific probes to detect isolates of further human IFNτ coding sequences and/or pseudogenes. Further, there may be more than one isoform of the IFNτ protein and more than one coding sequence per species. The specific nucleic acid probes used in the practice of the present invention and antibodies reactive with the IFNτ polypeptides of the present invention may be useful to isolate unidentified variants of interferon-τ in mammals, according to the methods of the invention disclosed herein.

### 2. Characterization of the Expression of Interferon-τ in Human Tissues.

Human placental cDNA libraries and an ovine cDNA library were analyzed by hybridization to the OvIFNτ cDNA probe (Example 8). This DNA hybridization analysis suggested that the IFNτ-signals from human cDNA libraries were approximately 1/100 of the signal obtained using the ovine cDNA library. OvIFNτ cDNAs constitute around 0.4% of the ovine cDNA library. Accordingly, the abundance of human cDNAs responding to the OvIFNτ probe appears to be low, at least in the term placenta from which the cDNA libraries were generated.

The presence of HuIFNτ mRNA in human term placenta and amniocytes were also analyzed. The results suggest the presence of human IFNτ mRNA in the feto-placental annex. The aminocytes also expressed the messages corresponding to OvIFNτ primers and human probe, suggesting that the expression of IFNτ mRNA is not limited to the term placenta.

In addition, a RT-PCR analysis for the presence of HuIFNτ was applied to the total cellular RNA isolated from human adult lymphocytes: the results suggest that IFNτ mRNA exists in lymphocytes.

The expression of interferon-τ in human tissue was also examined using *in situ* hybridization (Example 9). Sections from four healthy, different term and first trimester human placentas were examined. This analysis employed a cDNA probe derived from the OvIFNτ cDNA sequences (Example 9B). *In situ* hybridization was performed using an anti-sense RNA probe. In three separate experiments, specific hybridization was observed in all term and first trimester placental tissues.

First trimester placental villi (composed of an outer layer of syncytiotrophoblast, an underlying layer of cytotrophoblast, and a central stromal region with various types of mesenchymal cells) displayed the highest transcript level of IFNτ in the cytotrophoblast cells. Less intense but detectable levels were present in both the syncytiotrophoblast and stromal cells. A similar pattern of transcript expression was demonstrated in the placental villi of term tissue but the level of signal detection was low. First trimester extravillous trophoblasts displayed the highest amount of message and stained positive when present in the maternal blood spaces.

Howatson, et al., (1988) noted IFNα production in the syncytiotrophoblast of chorionic villi in both first trimester and term tissues. Also, Paulesu, *et al.* (1991) observed IFNα in extravillous trophoblast as well as syncytiotrophoblasts, noting more intense and abundant reactivity in first trimester placental tissue when compared to those taken at term. These investigators employed antibodies raised against human IFNα subtypes, and none observed any IFNα in the villous cytotrophoblasts.

The present results demonstrate that the human IFNτ gene is highly expressed in early placental tissues by migrating extravillous trophoblasts, but is also expressed in villous syncytiotrophoblasts, cytotrophoblasts, and various stromal cells. These results demonstrate the detection of IFNτ transcripts in human pregnancy tissues, and IFNτ expression in the villous cytotrophoblasts as well as the extravillous trophoblast of first trimester placenta.

### C. Antiviral Properties of Interferon-τ.

The antiviral activity of IFNτ has been evaluated against a number of viruses, including both RNA and DNA viruses. The relative specific activity of OvIFNτ, purified to homogeneity, was evaluated in antiviral assays (Example 10). IFNτ had a higher specific antiviral activity than either rBoIFNα or rBoIFNγ (Example 10, Table 3).

One advantage of the present invention is that IFNτ has potent antiviral activity with limited cytotoxic effects. Highly purified OvIFNτ was tested for anti-retroviral and cytotoxic effects on peripheral blood lymphocytes exposed to feline AIDS and human AIDS retroviruses (Bazer, F.W., *et al.,* (1989)). This feline AIDS lentivirus produces a chronic AIDS-like syndrome in cats and is a model for human AIDS (Pederson, *et al.,* 1987). Replication of either virus in peripheral blood lymphocytes (PBL) was monitored by reverse transcriptase (RT) activity in culture supernatants over time.

To determine IFNτ antiviral activity against FIV and HIV, RNA-dependent DNA polymerase RT activity was assayed in FIV- and HIV-infected feline and human PBL cultures treated with IFNτ (Example 11). Replication of FIV was reduced to about one-third of control values when cells were cultured in the presence of IFNτ. Addition of OvIFNτ produced a rapid, dose-dependent decrease in reverse transcriptase (RT) activity (Example 11, Table 4). While concentrations as low as 0.62 ng/ml of IFNτ inhibited viral replication, much higher concentrations (40 ng/ml) having greater effects on RT-activity were without toxic effects on the cells. The results suggest that replication of the feline immunodeficiency virus was reduced significantly compared to control values when cells were cultured in the presence of OvIFNτ.

IFNτ appeared to exert no cytotoxic effect on the cells hosting the retrovirus. This was true even when IFNτ was present at 40 ng per ml of culture medium. This concentration of IFNτ is equivalent to about 8,000 anti-viral units of alpha interferon, when IFNτ is assayed for its ability to protect Madin-Darby bovine kidney cells from lysis by vesicular stomatitis virus as described by Pontzer, et al. (1988).

IFNτ was also tested for activity against HIV replication in human cells. Human peripheral lymphocytes, which had been infected with HIV were treated with varying concentrations of IFNτ (Example 12). Replication of HIV in peripheral blood lymphocytes was monitored by reverse transcriptase activity in culture supernatants over time. Over a range of concentrations of IFNτ produced significant anti-HIV effects (Example 12, Table 5). A concentration of only 10 ng/ml resulted in over a 50% reduction in RT activity after only six days. A concentration of 500 ng/ml resulted in a 90% reduction in RT activity within 10 days. Further, there was no evidence of any cytotoxic effects attributable to the administration of IFNτ (Example 12, Table 5).

Further, the antiviral effects of IFNτ against HIV were evaluated by treating human PBMC cells with various amounts of either recombinant IFNτ or recombinant human IFNα₂ at the time of infection with HIV (Example 18). The data from these experiments (Example 18, Table 12) support the conclusion that, at similar concentrations, IFNα and IFNτ are effective in reducing the replication of HIV in human lymphocytes. However, treatment of cells with IFNα₂ resulted in cytoxicity, whereas no such cytotoxity was observed with treatment using IFNτ, even when IFNτ was used at much higher concentrations. No cytotoxicity was observed using IFNτ, even when IFNτ was used at 200 times the dosage of interferon-alpha II.

Both FIV and HIV reverse transcriptase themselves were unaffected by IFNτ in the absence of PBL. Therefore, the antiviral activity is not due to a direct effect on the viral RT.

Interferon-τ has also been shown to inhibit Hepatitis B Virus DNA replication in hepatocytes (Example 18). A human cell derived from liver cells transfected with Hepatitis B Virus (HBV) was used to test the antiviral effects of IFNτ. The cells were treated with both the IFNα and IFNτ over a range of concentrations. Both IFNα and IFNτ reduced DNA production by approximately two-fold compared to the no interferon control.

To demonstrate that the effect of the interferons was specific to the infecting virus and not the result of an effect on general cell metabolism, the hepatocyte was examined for the effects of IFNα and IFNτ on hepatospecific mRNA production (Example 18). Two hepatocyte specific proteins, Apo E and Apo A1, were detected by hybridization analysis. There was no apparent reduction of mRNA production for either hepatospecific mRNA at concentrations up to 40,000 units/ml of either IFNα or IFNτ. Further, no evidence for hepatotoxicity with IFNτ was seen in this assay.

These results suggest that IFNτ is an effective antiviral agent against a wide variety of viruses, including both RNA and DNA viruses. One advantage of IFNτ over other interferons, such as IFNα, is that treatment with IFNτ does not appear to be associated with any cytotoxicity.

### D. Antiproliferative Properties of IFNτ.

The effects of IFNτ on cellular growth have also been examined. In one analysis, anti-cellular growth activity was examined using a colony inhibition assay (Example 13). Human amnion (WISH) or MDBK cells were plated at low cell densities to form colonies originating from single cells. Dilutions of interferons were added to triplicate wells and the plates were incubated to allow colony formation. IFNτ inhibited both colony size and number in these assays. IFNτ was more effective at inhibiting cell proliferation of the human cell line (WISH) than human IFNα. The antiproliferative activity of IFNτ was dose-dependent. High concentrations of IFNτ stopped proliferation, while cell viability was not impaired.

Based on cell cycle analysis, using flow cytometry, IFNτ appears to inhibit progress of cells through S phase. These results demonstrate the antiproliferative effect of IFNτ, and underscore its low cytotoxicity.

The antiproliferative effects of IFNτ were also studied for rat and bovine cell lines (Example 14). The rate of ³H-thymidine incorporation was used to assess the rate of cellular proliferation. The data obtained demonstrate that IFNτ drastically reduced the rate of cellular proliferation (Example 14, Table 7) for each tested cell line.

The antiproliferative activity and lack of toxicity of IFNτ was further examined using a series of human tumor cell lines (Example 15). A variety of human tumor cell lines were selected from the standard lines used in NIH screening procedure for antineoplastic agents (Pontzer, C.H., *et al.*, (1991)). At least one cell line from each major neoplastic category was examined.
The following cell lines were obtained from American Type Culture Collection (12301 Parklawn Dr., Rockville MD 20852) :
- NCI-H460: human lung large cell carcinoma;
- DLD-1: human colon adenocarcinoma;
- SK-MEL-28: human malignant melanoma;
- ACHN: human renal adenocarcinoma;
- HL-60: human promyelocytic leukemia;
- H9: human T cell lymphoma;
- HUT 78: human cutaneous T cell lymphoma; and
- MCF7: human breast adenocarcinoma.

As above, the antiproliferative activity was evaluated by measuring the rate of ³H-thymidine incorporation into cells which have been treated with IFNτ. Significant differences between treatments were assessed by an analysis of variance followed by Scheffe's F-test. Cell cycle analysis was performed by flow cytometry.

Examination of IFNτ inhibition of MCF7 (breast adenocarcinoma) proliferation demonstrated that IFNτ reduced MCF7 proliferation in a dose-dependent manner. A 50% reduction in ³H-thymidine was observed with 10,000 units/ml of IFNτ (Example 15, Table 8). This cell line had previously been found to be unresponsive to anti-estrogen treatment.

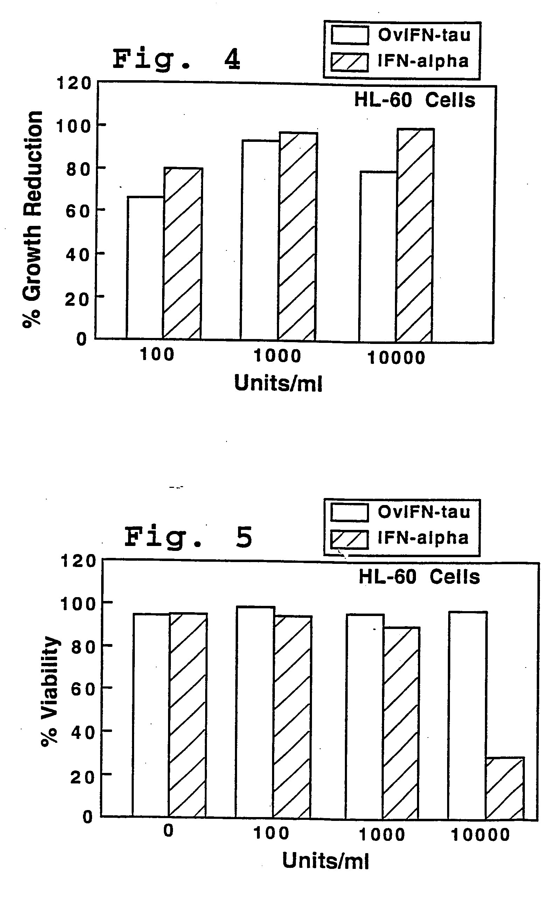

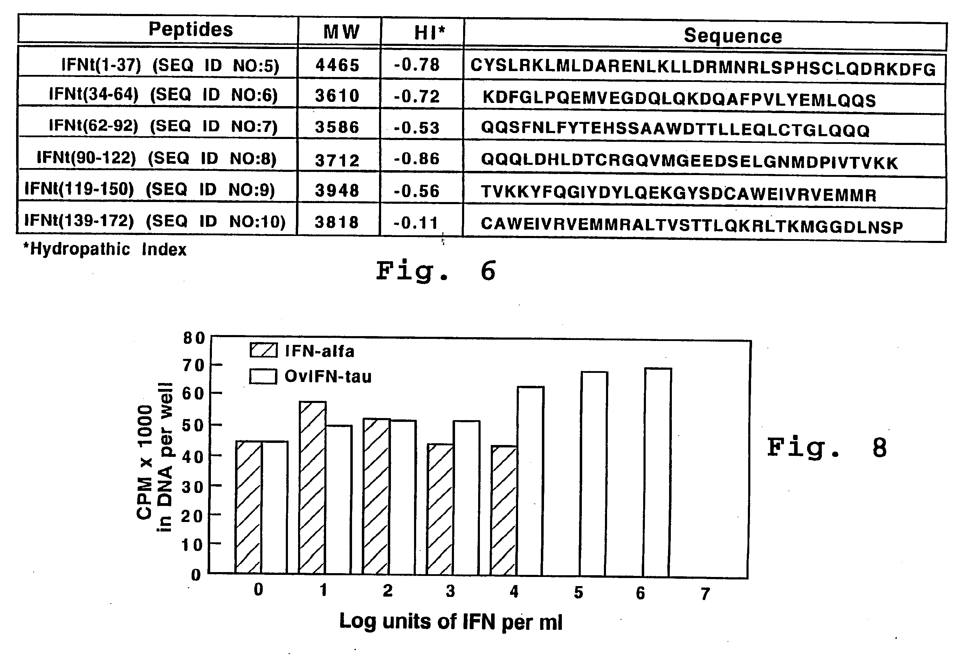

A comparison of the antiproliferative effects of IFNτ and IFNα was conducted using HL-60 (human promyelocytic leukemia) cells. Results with the promyelocytic leukemia HL-60 are typical of those obtained comparing IFNτ with human IFNα (Example 15). Concentrations as low as 100 units/ml of both IFNs produced significant (> 60%) growth reduction. Increasing amounts of IFNs further decreased tumor cell proliferation (Figure 4). At high doses of IFNα, but not IFNτ, was cytotoxic (Figure 5). Cell viability was reduced by approximately 80% by IFNα. By contrast, nearly 100% of the IFNτ-treated cells remained viable when IFNτ was applied at 10,000 units/ml. Thus, while both interferons inhibit proliferation, only IFNτ is without cytotoxicity. This lack of toxicity provides an advantage of IFNτ for use *in vivo* therapies.

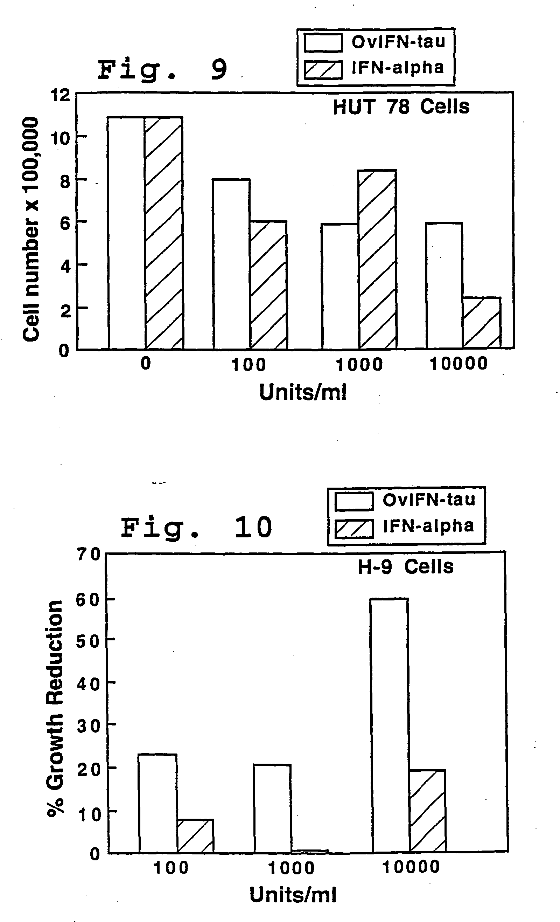

The human cutaneous T cell lymphoma, HUT 78, responded similarly to HL-60 when treated with IFNτ (Example 15, Figure 9). Both OvIFNτ and rHuIFNα reduce HUT 78 cell growth, but IFNα demonstrated adverse effects on cell viability.

The T cell lymphoma H9 was less sensitive to the antiproliferative effects of IFNα than the tumor cell lines described above. While IFNα was not toxic to the H9 cells, it failed to inhibit cell division significantly at any of the concentrations examined (Example 15, Figure 10). In contrast, IFNτ was observed to reduce H9 growth by approximately 60%. Thus, only OvIFNτ is an effective growth inhibitor of this T cell lymphoma.

In three additional tumor cell lines (NCI-H460, DLD-1 and SK-MEL-28) IFNτ and IFNα were equally efficacious antitumor agents. In the melanoma, SK-MEL-28, inhibition of proliferation by IFNα was accomplished by a 13% drop in viability, while IFNτ was not cytotoxic. In the majority of tumors examined, IFNτ is equal or preferable to IFNα as an antineoplastic agent against human tumors.

IFNτ exhibits antiproliferative activity against human tumor cells without toxicity and is as potent or more potent than human IFNα. Clinical trials of the IFNα2s have shown them to be effective antitumor agents (Dianzani, F., 1992; Krown, 1987). One therapeutic advantage of IFNτ as a therapeutic is the elimination of toxic effects seen with high doses IFNαs.

An additional application of the IFNτ is against tumors like Kaposi's sarcoma (associated with HIV infection) where the antineoplastic effects of IFNτ are coupled with IFNτ ability to inhibit retroviral growth.

The *in vivo* efficacy of interferon-τ treatment was examined in a mouse system (Example 16). B16-F10 is a syngeneic mouse transplantable tumor selected because of its high incidence of pulmonary metastases (Poste, *et al.,* 1981). Interferon treatment was initiated 3 days after the introduction of the tumor cells. The *in vivo* administration of IFNτ dramatically reduced B16-F10 pulmonary tumors. Thus, IFNτ appears to be an efficacious antineoplastic agent *in vivo* as well as *in vitro.*

### III. Interferon-τ Polypeptide Fragments, Protein Modeling and Protein Modifications.

The variety of IFNτ activities, its potency and lack of cytotoxicity, as taught by the present specification, suggest the importance of structure/function analysis for this novel interferon. The structural basis for OvIFNτ function has been examined using six overlapping synthetic peptides corresponding to the entire OvIFNτ sequence (Figure 6). The corresponding polypeptides derived from the ovine IFNτ sequence are presented as SEQ ID NO:15 to SEQ ID NO:20. Three peptides representing amino acids 1-37, 62-92 and 139-172 have been shown to inhibit IFNτ antiviral activity (Example 17). The peptides were effective competitors at concentrations of 300 µM and above.

The C-terminal peptide of IFNτ, OvIFNτ (139-172), and the internal peptide OvIFNτ (62-92), inhibited IFNτ and rBoIFNα_{II} antiviral activity to the same extent, while the N-terminal peptide OvIFNτ (1-37) was more effective in inhibiting OvIFNτ antiviral activity. Dose-response data indicated that IFNτ (62-92) and IFNτ (139-172) inhibited IFNτ antiviral activity to similar extents. The same peptides that blocked IFNτ antiviral activity also blocked the antiviral activity of recombinant bovine IFNα (rBoIFNα); recombinant bovine IFNγ was unaffected by the peptides. These two IFNτ peptides may represent common receptor binding regions for IFNτ and various IFNαs.

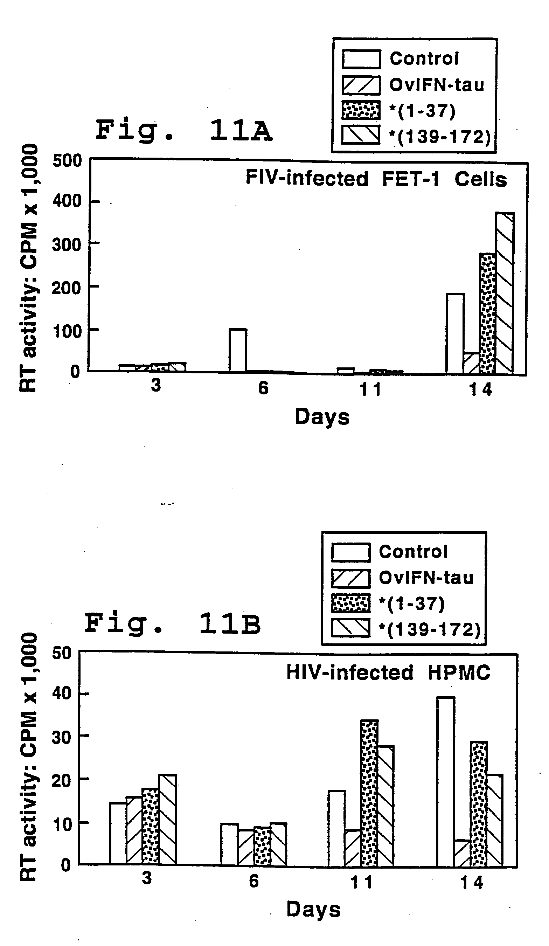

The two synthetic peptides OvIFNτ(1-37) and OvIFNτ(139-172) also blocked OvIFNτ anti-FIV and anti-HIV activity (Example 17; Figures 11A and 11B). While both peptides blocked FIV RT activity, only the C-terminal peptide, OvIFNτ(139-172), appeared to be an efficient inhibitor of vesicular stomatitis virus activity on the feline cell line, Fc9.

The above data taken together suggest that the C-terminal regions of type I interferons may bind to common site on the type I interferon receptor, while the N-terminal region may be involved in the elicitation of unique functions. These results suggest that portions of the IFNτ interferon molecule may be used to substitute regions of interferon alpha molecules. For example, the region of an interferon alpha molecule that is responsible for increased cytotoxicity, relative to IFNτ treatment, can be identified by substituting polypeptide regions derived from IFNτ for regions of an interferon alpha molecule. Such substitutions can be carried out by manipulation of synthetic genes (see below) encoding the selected IFNτ and interferon alpha molecules, coupled to the functional assays described herein (such as, antiviral, antiproliferative and cytoxicity assays).

Polyclonal anti-peptide antisera against the IFNτ peptides yielded similar results as the polypeptide inhibition studies, described above. Antibodies directed against the same three regions (OvIFNτ (1-37), IFNτ (62-92) and IFNτ (139-172)) blocked OvIFNτ function, confirming the importance of these three domains in antiviral activity (Example 17). These peptides, although apparently binding to the interferon receptor, did not in and of themselves elicit interferon-like effects in the cells.

The antiproliferative activity of IFNτ (Example 17, Table 11) involved a further region of the molecule, since IFNτ(119-150) was the most effective inhibitor of OvIFNτ-induced reduction of cell proliferation. This results suggests that the region of the molecule primarily responsible for inhibition of cell growth is the IFNτ(119-150) region. This region of the IFNτ molecule may be useful alone or fused to other proteins (such as serum albumin, an antibody or an interferon alpha polypeptide) as an antineoplastic agent. A conjugated protein between an N-terminal peptide derived from human interferon-α and serum albumin was shown to have anticellular proliferation activity (Ruegg, et al., 1990).

Finally, binding of ¹²⁵I-OvIFNτ to its receptor on MDBK cells could be blocked by antisera to 4 of the 6 peptides; the 4 polypeptides representing amino acids 1-37, 62-92, 119-150 and 139-172 of OvIFNτ. This reflects the multiple binding domains as well as the functional significance of these regions. Since different regions of IFNτ are involved in elicitation of different functions, modification of selected amino acids could potentially result in IFNτ-like interferons with selective biological activity.

The above data demonstrate the identification of synthetic peptides having four discontinuous sites on the OvIFNτ protein that are involved in receptor interaction and biological activity. In order to elucidate the structural relationship of these regions, modeling of the three dimensional structure of IFNτ was undertaken. A three dimensional model would be useful in interpretation of existing data and the design of future structure/function studies.

Combining circular dichroism (CD) of both the full length recombinant OvIFNτ to IFNβ (a protein of known three dimensional structure (Senda, *et al.,* 1992)), a model of OvIFNτ has been constructed. The most striking feature of this model is that IFNτ falls into a class of proteins with a four-helix bundle motif. The CD spectra of IFNτ was taken on an AVIV 60 S spectropolarimeter. Two different methods were employed for secondary structure estimations, the algorithm of Perczel, *et al.*, (1991) and variable selection by W.C. Johnson, Jr. (1992).

Secondary structure estimations of the spectra indicate 70-75% alpha helix (characterized by minima at 222 and 208 nm and maximum at 190 nm). The variable selection algorithm estimates the remainder of the molecule to be 20% beta sheet and 10% turn. The Chang method estimates the remainder to be 30% random coil. Alignment of IFNτ and IFNβ sequences revealed homology between the two molecules, specifically in the regions of known helical structure in IFNβ. Sequence analysis of IFNτ also showed that proposed helical regions possess an apolar periodicity indicative of a four-helix bundle motif.

The final modeling step was to apply the IFNβ x-ray crystallographic coordinates of the IFNβ carbon backbone to the IFNτ sequence. The functionally active domains of IFNτ, identified above, were localized to one side of the molecule and found to be in close spatial proximity. This is consistent with multiple binding sites on IFNτ interacting simultaneously with the type I IFN receptor.

The three dimensional modeling data coupled with the function data described above, provides the ability to introduce sequence variations into specific regions of IFNτ to generate enhancement of selected functions (e.g., antiviral or anticellular proliferation) or the ability to substitute a region(s) of selected function into other interferon molecules (e.g., antiviral, antineoplastic, or reduced cytotoxicity).

The construction of a synthetic gene for OvIFNτ is described in Example 3. Briefly, a consensus amino sequence was back-translated using optimal codon usage for *E. coli.* The sequence was edited to include 20, unique, restriction sites spaced throughout the length of the construct. This 540 base pair synthetic gene sequence was divided into 11 oligonucleotide fragments. Individual fragments were synthesized and cloned, either single or double stranded, into either pTZ 19R, pTZ 18R or pBluescript, amplified and fused. The synthetic OvIFNτ construct was then cloned into a modified pIN-III-ompA expression vector for expression in bacteria and also cloned into a yeast expression plasmid. A similarly constructed human IFNτ synthetic gene (SEQ ID NO:3) has been designed, constructed and expressed in yeast cells.

Expression of the OvIFNτ synthetic gene in yeast (Example 4) allowed over production of recombinant IFNτ in *S. cerevisiae:* large quantities (5-20 mg/l) of recombinant IFNτ can be purified from soluble yeast extract using sequential ion exchange and molecular sieve chromatography. Recombinant IFNτ purified in this fashion exhibited potent antiviral activity (2 to 3 X 10⁸ units/mg) similar to native OvIFNτ.

The synthetic gene construct facilitates introduction of mutations for possible enhancement of antitumor (anticellular proliferative) and antiviral activities. Further, the disparate regions of the molecule responsible for different functions allows for separate manipulation of different functions. For example, two deletion mutants, OvIFNτ(1-155) and OvIFNτ(1-166), have been constructed to examine the role of carboxy terminal sequences in IFNτ molecules.

Additional mutant IFNτ molecules have been constructed to identify residues critical for antiproliferative activity. For example, one particular residue, Tyr 123 has been implicated in the anticellular proliferative activity of IFNα (McInnes, *et al.,* 1989). The equivalent of Tyr 123 in IFNτ is contained within peptide OvIFNτ(119-150): this polypeptide inhibits OvIFNτ and human IFNα antiproliferative activity. Mutations converting Tyr 123 to conservative (Trp) and nonconservative (Asp) substitutions have been generated, as well as mutant sequences having deletion of this residue. The codon for Tyr 123 is located within an SspI site; elimination of this site has been used for screening. The antiproliferative activity of these mutant IFNτ is evaluated as described herein.

Synthetic peptides can be generated which correspond to the IFNτ polypeptides of the present invention. Synthetic peptides can be commercially synthesized or prepared using standard methods and apparatus in the art (Applied Biosystems, Foster City CA).

Alternatively, oligonucleotide sequences encoding peptides can be either synthesized directly by standard methods of oligonucleotide synthesis, or, in the case of large coding sequences, synthesized by a series of cloning steps involving a tandem array of multiple oligonucleotide fragments corresponding to the coding sequence (Crea; Yoshio *et al.;* Eaton *et al.).* Oligonucleotide coding sequences can be expressed by standard recombinant procedures (Maniatis *et al.;* Ausubel *et al.).*

The biological activities of the interferon-τ polypeptides described above can be exploited using either the interferon-τ polypeptides alone or conjugated with other proteins (see below).

### IV. Production of Fusion Proteins.

In another aspect, the present invention includes interferon-τ or interferon-τ-derived polypeptides covalently attached to a second polypeptide to form a fused, or hybrid, protein. The interferon-τ sequences making up such fused proteins can be recombinantly produced interferon-τ or a bioactive portion thereof, as described above.

For example, where interferon-τ is used to inhibit viral expression, the polypeptides presented as SEQ ID NO:10 and SEQ ID NO:20 may be advantageously fused with a soluble peptide, such as, serum albumin, an antibody (*e.g*., specific against an virus-specific cell surface antigen), or an interferon alpha polypeptide. Other examples of fusion proteins include (i) replacing toxicity-associated regions of interferon-α with the interferon-τ regions SEQ ID NO:5 and SEQ ID NO:15, and (ii) fusion proteins containing the interferon-τ regions SEQ ID NO:9 and SEQ ID NO:19 as anticellular proliferation agents.

The fused proteins of the present invention may be formed by chemical conjugation or by recombinant techniques. In the former method, the interferon-τ and second selected polypeptide are modified by conventional coupling agents for covalent attachment. In one exemplary method for coupling soluble serum albumin to an interferon-τ polypeptide, serum albumin is derivatized with N-succinimidyl-S-acetyl thioacetate (Duncan), yielding thiolated serum albumin. The activated serum albumin polypeptide is then reacted with interferon-τ derivatized with N-succinimidyl 3-(2-pyridyldithio) propionate (Cumber), to produce the fused protein joined through a disulfide linkage.

As an alternative method, recombinant interferon-τ may be prepared with a cysteine residue to allow disulfide coupling of the interferon-τ to an activated ligand, thus simplifying the coupling reaction. An interferon-τ expression vector, used for production of recombinant interferon-τ, can be modified for insertion of an internal or a terminal cysteine codon according to standard methods of site-directed mutagenesis (Ausubel, *et al*.).

In one method, a fused protein is prepared recombinantly using an expression vector in which the coding sequence of a second selected polypeptide is joined to the interferon-τ coding sequence. For example, human serum albumin coding sequences can be fused in-frame to the coding sequence of an interferon-τ polypeptide, such as, SEQ ID NO:9. The fused protein is then expressed using a suitable host cell. The fusion protein may be purified by molecular-sieve and ion-exchange chromatography methods, with additional purification by polyacrylamide gel electrophoretic separation and/or HPLC chromatography, if necessary.

It will be appreciated from the above how interferon-τ-containing fusion proteins may be prepared. One variation on the above fusion is to exchange positions of the interferon-τ and selected second protein molecules in the fusion protein *(e.g.*, carboxy terminal versus amino terminal fusions). Further, internal portions of a native interferon-τ polypeptide (for example, amino acid regions of between 15 and 172 amino acids) can be assembled into polypeptides where two or more such interferon-τ portions are contiguous that are normally discontinuous in the native protein.

### V. Antibodies Reactive with Interferon-τ.

Fusion proteins containing the polypeptide antigens of the present invention fused with the glutathione-S-transferase (Sj26) protein can be expressed using the pGEX-GLI vector system in *E. coli* JM101 cells. The fused Sj26 protein can be isolated readily by glutathione substrate affinity chromatography (Smith). Expression and partial purification of IFNτ proteins is described in (Example 20), and is applicable to any of the other soluble, induced polypeptides coded by sequences described by the present invention.

Insoluble GST (sj26) fusion proteins can be purified by preparative gel electrophoresis.

Alternatively, IFNτ-β-galactosidase fusion proteins can be isolated as described in Example 19.

Also included in the invention is an expression vector, such as the lambda gtl1 or pGEX vectors described above, containing IFNτ coding sequences and expression control elements which allow expression of the coding regions in a suitable host. The control elements generally include a promoter, translation initiation codon, and translation and transcription termination sequences, and an insertion site for introducing the insert into the vector.

The DNA encoding the desired polypeptide can be cloned into any number of vectors (discussed above) to generate expression of the polypeptide in the appropriate host system. These recombinant polypeptides can be expressed as fusion proteins or as native proteins. A number of features can be engineered into the expression vectors, such as leader sequences which promote the secretion of the expressed sequences into culture medium. Recombinantly produced IFNτ, and polypeptides derived therefrom, are typically isolated from lysed cells or culture media. Purification can be carried out by methods known in the art including salt fractionation, ion exchange chromatography, and affinity chromatography. Immunoaffinity chromatography can be employed using antibodies generated against selected IFNτ antigens.

In another aspect, the invention includes specific antibodies directed against the polypeptides of the present invention. Typically, to prepare antibodies, a host animal, such as a rabbit, is immunized with the purified antigen or fused protein antigen. Hybrid, or fused, proteins may be generated using a variety of coding sequences derived from other proteins, such as β-galactosidase or glutathione-S-transferase. The host serum or plasma is collected following an appropriate time interval, and this serum is tested for antibodies specific against the antigen. Example 20 describes the production of rabbit serum antibodies which are specific against the IFNτ antigens in a Sj26/IFNτ hybrid protein. These techniques can be applied to the all of the IFNτ molecules and polypeptides derived therefrom.

The gamma globulin fraction or the IgG antibodies of immunized animals can be obtained, for example, by use of saturated ammonium sulfate or DEAE Sephadex, or other techniques known to those skilled in the art for producing polyclonal antibodies.

Alternatively, purified protein or fused protein may be used for producing monoclonal antibodies. Here the spleen or lymphocytes from a animal immunized with the selected polypeptide antigen are removed and immortalized or used to prepare hybridomas by methods known to those skilled in the art (Harlow, *et al.).* Lymphocytes can be isolated from a peripheral blood sample. Epstein-Barr virus (EBV) can be used to immortalize human lymphocytes or a fusion partner can be used to produce hybridomas.

Antibodies secreted by the immortalized cells are screened to determine the clones that secrete antibodies of the desired specificity, for example, by using the ELISA or Western blot method (Ausubel *et al*.). Experiments performed in support of the present invention have yielded four hybridomas producing monoclonal antibodies specific for ovine IFNτ have been isolated.

Antigenic regions of polypeptides are generally relatively small, typically 7 to 10 amino acids in length. Smaller fragments have been identified as antigenic regions. Interferon-τ polypeptide antigens are identified as described above. The resulting DNA coding regions can be expressed recombinantly either as fusion proteins or isolated polypeptides.

In addition, some amino acid sequences can be conveniently chemically synthesized (Applied Biosystems, Foster City CA). Antigens obtained by any of these methods may be directly used for the generation of antibodies or they may be coupled to appropriate carrier molecules. Many such carriers are known in the art and are commercially available (*e.g*., Pierce, Rockford IL).

Antibodies reactive with IFNτ are useful, for example, in the analysis of structure/function relationships.

### VI. Utility

### A. Reproductive.

Although IFNτ bears some similarity to the IFNα family based on structure and its potent antiviral properties, the IFNαs do not possess the reproductive properties associated with IFNτ. Also, recombinant bovine IFNα has little or no effect on interestrous interval compared to IFNτ (Davis, *et al.,* 1992).

Therefore, although IFNτ has some structural similarities to other interferons, it has very distinctive properties of its own: for example, the capability of significantly influencing the biochemical events of the estrous cycle.

The human IFNτ of the present invention can be used in methods of enhancing fertility and prolonging the life span of the *corpus luteum* in female mammals as generally described in Hansen, *et al.,* herein incorporated by reference. Further, the human interferon-τ of the present invention could be used to regulate growth and development of uterine and/or fetal-placental tissues. The human IFNτ is particularly useful for treatment of humans, since potential antigenic responses are less likely using such a same-species protein.

### B. Antiviral Properties.

The antiviral activity of IFNτ has broad therapeutic applications without the toxic effects that are usually associated with IFNαs. Although the presence of IFNτ in culture medium inhibited reverse transcriptase activity of the feline immunodeficiency virus (Example 11), this is not due to a direct effect of IFNτ on the FIV. Rather, IFNτ appears to induce the host cell to produce a factor(s) which is inhibitory to the reverse transcriptase of the virus.

IFNτ was found to exert its antiviral activity without adverse effects on the cells: no evidence of cytotoxic effects attributable to the administration of IFNτ was observed. It is the lack of cytotoxicity of IFNτ which makes it extremely valuable as an *in vivo* therapeutic agent. This lack of cytotoxicity sets IFNτ apart from most other known antiviral agents and all other known interferons.

Formulations comprising the IFNτ compounds of the present invention can be used to inhibit viral replication.

The human IFNτ of the present invention can be employed in methods for affecting the immune relationship between fetus and mother, for example, in preventing transmission of maternal viruses (*e.g*., HIV) to the developing fetus. The human interferon-τ is particularly useful for treatment of humans, since potential antigenic responses are less likely using a homologous protein.

### C. Anticellular Proliferation Properties.

IFNτ exhibits potent anticellular activity. IFNτ can also be used to inhibit cellular growth without the negative side effects associated with other interferons which are currently known. Formulations comprising the IFNτ compounds of the subject invention can be used to inhibit, prevent, or slow tumor growth.

The development of certain tumors is mediated by estrogen. Experiments performed in support of the present invention indicate that IFNτ can suppress estrogen receptor numbers. Therefore, IFNτ can be used in the treatment or prevention of estrogen-dependent tumors.

### D. Interfering with the Binding of Interferons to Receptors.

IFNτ appears to interact with the Type I IFN receptor via several epitopes on the molecule, and these regions either separately or in combination may affect distinct functions of IFNτ differently.

The polypeptides of the present invention are useful for the selective inhibition of binding of interferons to the interferon receptor. Specifically, as described herein, certain of the disclosed peptides selectively inhibit the antiviral activity of IFNτ while others inhibit the antiproliferative activity. Combinations of these peptides could be used to inhibit both activities. Advantageously, despite binding to the interferon receptor and blocking IFNτ activity, these peptides do not, themselves, elicit the antiviral or antiproliferative activity.

Therefore, such polypeptides can be used as immunoregulatory molecules when it is desired to prevent immune responses triggered by interferon molecules. These peptides could be used as immunosuppressants to prevent, for example, interferon-mediated immune responses to tissue transplants. Other types of interferon mediated responses may also be blocked, such as the cytotoxic effects of alpha interferon.

### E. Pharmaceutical Compositions.

IFNτ proteins can be formulated according to known methods for preparing pharmaceutically useful compositions. Formulations comprising interferons or interferon-like compounds have been previously described (for example, Martin, 1976). In general, the compositions of the subject invention will be formulated such that an effective amount of the IFNτ is combined with a suitable carrier in order to facilitate effective administration of the composition.

The compositions used in these therapies may also be in a variety of forms. These include, for example, solid, semi-solid, and liquid dosage forms, such as tablets, pills, powders, liquid solutions or suspensions, liposomes, suppositories, injectable, and infusible solutions. The preferred form depends on the intended mode of administration and therapeutic application. The compositions also preferably include conventional pharmaceutically acceptable carriers and adjuvants which are known to those of skill in the art. Preferably, the compositions of the invention are in the form of a unit dose and will usually be administered to the patient one or more times a day.

IFNτ, or related polypeptides, may be administered to a patient in any pharmaceutically acceptable dosage form, including intravenous, intramuscular, intralesional, or subcutaneous injection. Specifically, compositions and methods used for other interferon compounds can be used for the delivery of these compounds.

One primary advantage of the compounds of the subject invention, however, is the extremely low cytotoxicity of the IFNτ proteins. Because of this low cytotoxicity, it is possible to administer the IFNτ in concentrations which are greater than those which can generally be utilized for other interferon (*e.g*., IFNα) compounds. Thus, IFNτ can be administered at rates from about 5 × 10⁴ to 20 × 10⁶ units/day to about 500 × 10⁶ units/day or more. In a preferred embodiment, the dosage is about 10⁶ units/day. High doses are preferred for systemic administration. It should, of course, be understood that the compositions and methods of this invention may be used in combination with other therapies.

Once improvement of a patient's condition has occurred, a maintenance dose is administered if necessary. Subsequently, the dosage or the frequency of administration, or both, may be reduced, as a function of the symptoms, to a level at which the improved condition is retained. When the symptoms have been alleviated to the desired level, treatment should cease. Patients may, however, require intermittent treatment on a long-term basis upon any recurrence of disease symptoms.

The compositions of the subject invention can be administered through standard procedures to treat a variety of cancers and viral diseases including those for which other interferons have previously shown activity. See, for example, Finter, *et al*. (1991); Dianzani, *et al*. (1992); Francis, *et al*. (1992) and U.S. Patent Nos. 4,885,166 and 4,975,276. However, as discussed above, the compositions of the subject invention have unique features and advantages, including their ability to treat these conditions without toxicity.

### F. Treatment of Skin Disorders.

Disorders of the skin can be treated intralesionally using IFNτ, wherein formulation and dose will depend on the method of administration and on the size and severity of the lesion to be treated. Preferred methods include intradermal and subcutaneous injection. Multiple injections into large lesions may be possible, and several lesions on the skin of a single patient may be treated at one time. The schedule for administration can be determined by a person skilled in the art. Formulations designed for sustained release can reduce the frequency of administration.

### G. Systemic Treatment.

Systemic treatment is essentially equivalent for all applications. Multiple intravenous or subcutaneous doses are possible, and in the case of implantable methods for treatment, formulations designed for sustained release are particularly useful. Patients may also be treated using implantable subcutaneous portals, reservoirs, or pumps.

### H. Regional Treatment.

Regional treatment with the IFNτ polypeptides of the present invention is useful for treatment of cancers in specific organs. Treatment can be accomplished by intraarterial infusion. A catheter can be surgically or angiographically implanted to direct treatment to the affected organ. A subcutaneous portal, connected to the catheter, can be used for chronic treatment, or an implantable, refillable pump may also be employed.

The following examples illustrate, but in no way are intended to limit the present invention.

### Materials and Methods

Restriction endonucleases, T4 DNA ligase, T4 polynucleotide kinase, *Taq* DNA polymerase, and calf intestinal phosphatase were purchased from New England Biolabs (Beverly, MA) or Promega Biotech (Madison, WI): these reagents were used according to the manufacturer's instruction. For sequencing reactions, a "SEQUENASE DNA II" sequencing kit was used (United States Biochemical Corporation, Cleveland OH). Immunoblotting and other reagents were from Sigma Chemical Co. (St. Louis, MO) or Fisher Scientific (Needham, MA). Nitrocellulose filters are obtained from Schleicher and Schuell (Keene, NH).

Synthetic oligonucleotide linkers and primers are prepared using commercially available automated oligonucleotide synthesizers (*e.g*., an ABI model 380B-02 DNA synthesizer (Applied Biosystems, Foster City, CA)). Alternatively, custom designed synthetic oligonucleotides may be purchased, for example, from Synthetic Genetics (San Diego, CA). cDNA synthesis kit and random priming labeling kits are obtained from Boehringer-Mannheim Biochemical (BMB, Indianapolis, IN).

Oligonucleotide sequences encoding polypeptides can be either synthesized directly by standard methods of oligonucleotide synthesis, or, in the case of large coding sequences, synthesized by a series of cloning steps involving a tandem array of multiple oligonucleotide fragments corresponding to the coding sequence (Crea; Yoshio et al.; Eaton et al.). Oligonucleotide coding sequences can be expressed by standard recombinant procedures (Maniatis et al.; Ausubel et al.).

Alternatively, peptides can be synthesized directly by standard *in vitro* techniques (Applied Biosystems, Foster City CA).

Common manipulations involved in polyclonal and monoclonal antibody work, including antibody purification from sera, are performed by standard procedures (Harlow et al.). Pierce (Rockford, IL) is a source of many antibody reagents.

Recombinant human IFNα (rHuIFNα) and rBoIFNγ was obtained from Genentech Inc. (South San Francisco, CA). The reference preparation of recombinant human IFNα (rHuIFNα) was obtained from the National Institutes of Health: rHuIFNα is commercially available from Lee Biomolecular (San Diego, CA).

All tissue culture media, sera and IFNs used in this study were negative for endotoxin, as determined by assay with Limulus amebocyte lysate (Associates of Cape Cod, Woods Hole, MA) at a sensitivity level of 0.07 ng/ml.

### General ELISA Protocol for Detection of Antibodies.

Polystyrene 96 well plates Immulon II (PGC) were coated with 5 µg/mL (100 µL per well) antigen in 0.1 M carb/bicarbonate buffer, pH 9.5. Plates were sealed with parafilm and stored at 4°C overnight.

Plates were aspirated and blocked with 300 uL 10% NGS and incubated at 37°C for 1 hr.

Plates were washed 5 times with PBS 0.5% "TWEEN-20".

Antisera were diluted in 0.1 M PBS, pH 7.2. The desired dilution(s) of antisera (0.1 mL) were added to each well and the plate incubated 1 hours at 37°C. The plates was then washed 5 times with PBS 0.5% "TWEEN-20".

Horseradish peroxidase (HRP) conjugated goat antihuman antiserum (Cappel) was diluted 1/5,000 in PBS. 0.1 mL of this solution was added to each well. The plate was incubated 30 min at 37°C, then washed 5 times with PBS.

Sigma ABTS (substrate) was prepared just prior to addition to the plate.

The reagent consists of 50 mL 0.05 M citric acid, pH 4.2, 0.078 mL 30% hydrogen peroxide solution and 15 mg ABTS. 0.1 mL of the substrate was added to each well, then incubated for 30 min at room temperature. The reaction was stopped with the addition of 0.050 mL 5% SDS (w/v). The relative absorbance is determined at 410 nm.

### EXAMPLE 1

### Reproductive Functions of IFNτ

The effect of interferon-τ on the lifespan of the corpus lutem was examined.

IFNτ was infused into uterine lumen of ewes at the concentrations given in Table 1. Recombinant human IFNα (rHuIFNα) was infused at similar concentrations. In addition, control animals, which received control proteins, were also used. The life span of the corpus luteum was assessed by examination of interestrous intervals, maintenance of progesterone secretion, and inhibition of prostaglandin secretion (Davis, *et al*., *1992*).

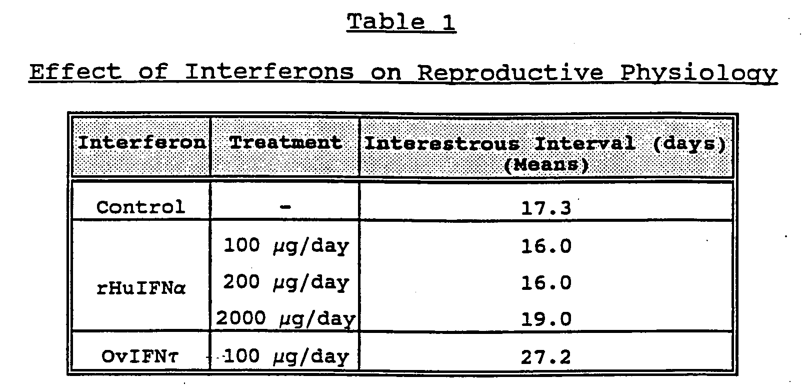

Comparison of the interestrous intervals for the control animals and for animals receiving OvIFNτ demonstrate a considerable lengthening of the interval, when IFNτ is administered at 100µg/day. On the other hand, comparison of the interestrous interval for the control animal and for animals receiving recombinant human IFNα, demonstrated that rHuIFNα had no meaningful effect.

These results demonstrate that interferon-τ has the capability of significantly influencing the biochemical events of the reproductive cycle.

### EXAMPLE 2

### Antiviral Properties of Interferon-τ at Various Stages of the Reproductive Cycle

Conceptus cultures were established using conceptus obtained from sheep at days 12 through 16 of the estrous cycle. Antiviral activity of supernatant from each conceptus culture was assessed using a cytopathic effect assay (Familetti, *et al.,* 1981). Briefly, dilutions of IFNτ or other IFNs were incubated with Madin-Darby bovine kidney (MDBK) cells for 16-18 hours at 37°C. Following incubation, inhibition of viral replication was determined in a cytopathic effect assay using vesicular stomatitis virus (VSV) as the challenge virus.

One antiviral unit caused a 50% reduction in destruction of the monolayer, relative to untreated MDBK cells infected with VSV (control plates). Specific activities were further evaluated using normal ovine fibroblasts (Shnf) in a plaque inhibition assay (Langford, *et al.,* 1981). A minimum of three samples were examined at each time point, and each sample was assayed in triplicate. The results presented in Table 2 are expressed as mean units/ml.

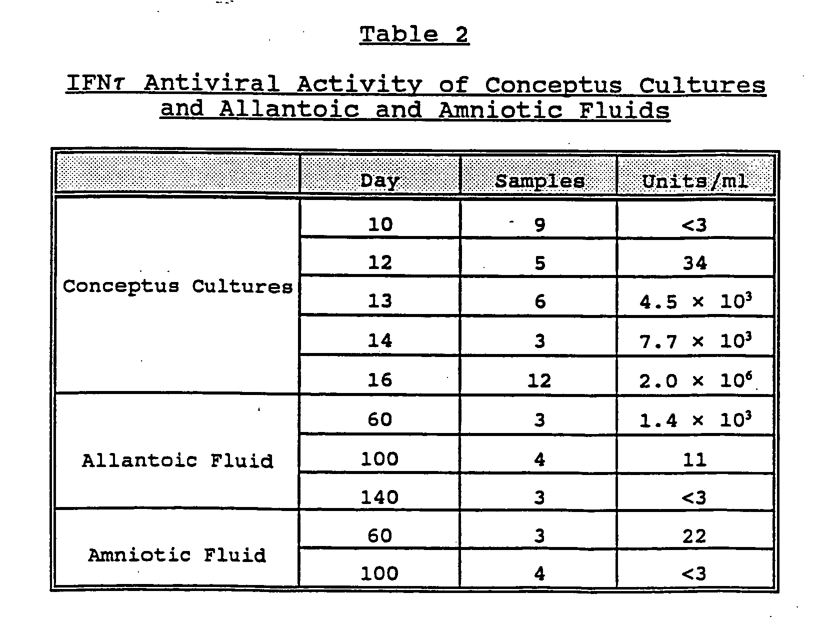

Culture supernatants had increasing antiviral activity associated with advancing development of the conceptus (Table 2).

### EXAMPLE 3

### Expression of IFNτ in Bacteria

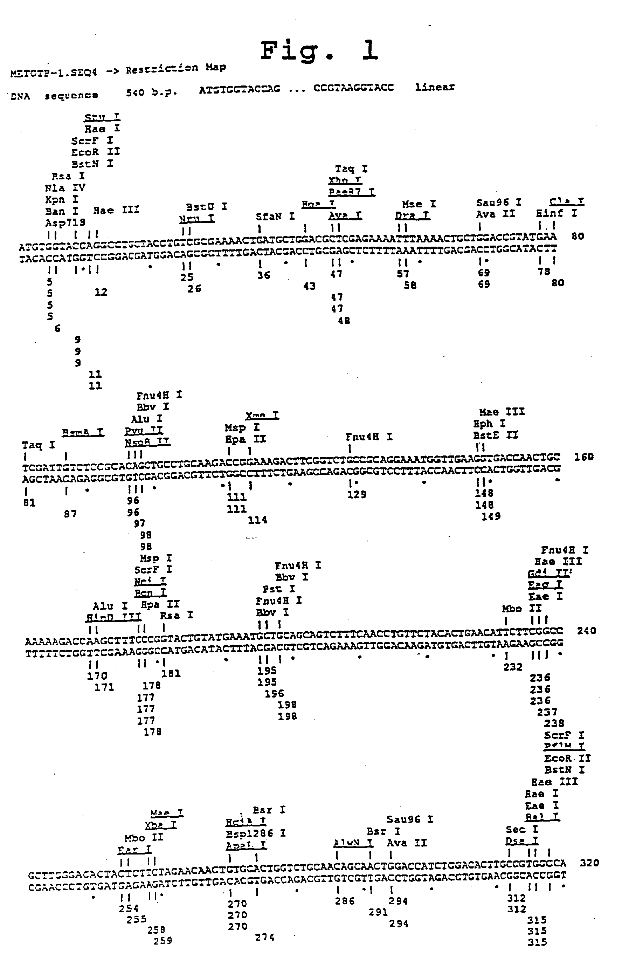

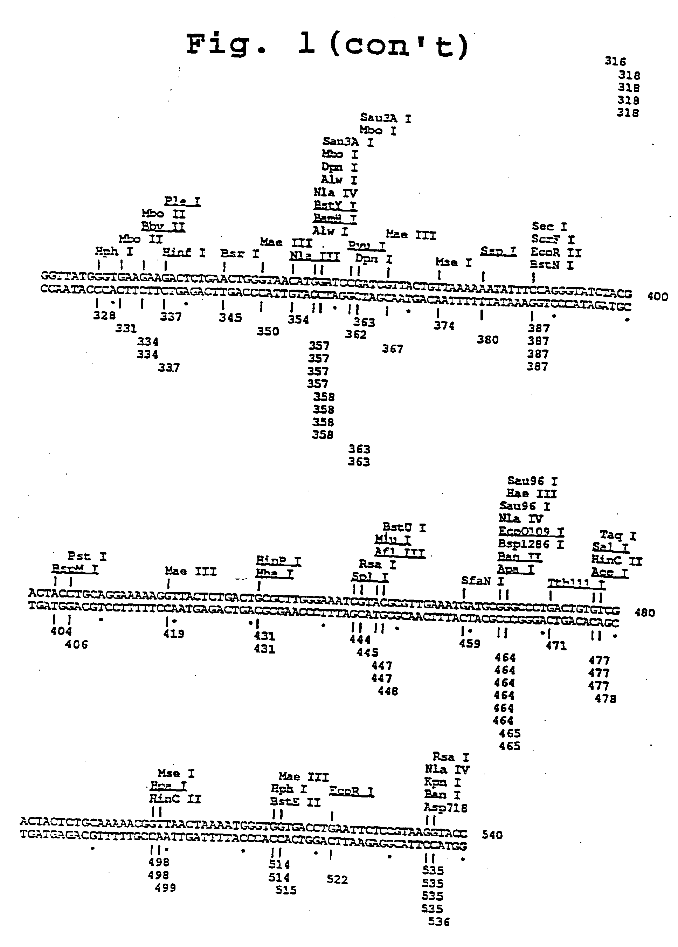

The amino acid coding sequence for OvIFNτ (Imakawa, *et al.,* 1987) was used to generate a corresponding DNA coding sequence with codon usage optimized for expression in *E. coli.* Linker sequences were added to the 5' and 3' ends to facilitate cloning in bacterial expression vectors. The nucleotide sequence was designed to include 19 unique restriction enzyme sites spaced evenly throughout the coding sequence (Figure 1).

The nucleotide sequence was divided into eleven oligonucleotide fragments ranging in sizes of 33 to 75 bases. Each of the eleven oligonucleotides were synthesized on a 380-B 2-column DNA synthesizer (Applied Biosytems) and cloned single- or double-stranded into one of the following vectors: "pBLUESCRIPT⁺(KS)" (Stratagene, LaJolla, CA), pTZ18R (Pharmacia, Piscataway, NJ), or PTZ19R (Pharmacia, Piscataway, NJ) cloning vectors.

The vectors were transformed into *E. coli* K. strain "XL1-BLUE" (recA1 endA1 gyrA96 thi hsdR17 (r_{*k*}⁻, m_{*k*}+) supE44 relA1 λ - (lac), {F', proAB, lac^{q}ZΔM15, Tn10(tet^{R}}) which is commercially available from Stratagene (LaJolla, CA). Transformed cells were grown in L broth supplemented with ampicillin (50 µg/ml). Oligonucleotide cloning and fusion was performed using standard recombinant DNA techniques.

Cloning vectors were cut with the appropriate restriction enzymes to insert the synthetic oligonucleotides. The vectors were treated with calf intestine alkaline phosphatase (CIP) to remove terminal phosphate groups. Oligonucleotides were phosphorylated and cloned, as either single- or double-stranded molecules, into the appropriate vector using T4 DNA ligase. When single-strands were introduced into cloning vectors, the second strand was completed by the bacterial host following transfection.

For double-stranded cloning, oligonucleotides were first annealed with their synthetic complementary strand then ligated into the cloning vector. *E. coli* K12 strains SB221 or NM522 were then transformed with the ligation. *E. coli* strain GM119 was used for cloning when the methylation-sensitive *Stu*I and *Cla*I restriction sites were involved. Restriction analyses were performed on isolated DNA at each stage of the cloning procedure.

Cloned oligonucleotides were fused into a single polynucleotide using the restriction digestions and ligations outlined in Figure 2. Oligonucleotide-containing-DNA fragments were typically isolated after electrophoretic size fractionation on low-melting point agarose gels (Maniatis, *et al*.; Sambrook, *et al*.; Ausubel, *et al*.). The resulting IFNτ polynucleotide coding sequence spans position 16 through 531: a coding sequence of 172 amino acids.

The nucleotide sequence of the final polynucleotide was confirmed by DNA sequencing using the dideoxy chain termination method.

The full length *Stu*I/*Sst*I fragment (540 bp; Figure 2) was cloned into a modified pIN III omp-A expression vector and transformed into a competent SB221 strain of *E. coli.* For expression of the IFNτ protein, cells carrying the expression vector were grown in L-broth containing ampicillin to an OD (550 nm) of 0.1-1, induced with IPTG for 3 hours and harvested by centrifugation. Soluble recombinant IFNτ was liberated from the cells by sonication or osmotic fractionation.

### EXAMPLE 4

### Expression of IFNτ in Yeast

The synthetic IFNτ gene, synthesized in Example 3, was flanked at the 5' end by an *StuI* restriction site and at the 3' end by a *SacI* restriction site.

### A. Isolation of the Synthetic IFNτ Gene.

Two oligonucleotide primers (SEQ ID NO:13 and SEQ ID NO:14) were used to attach linkers to the synthetic IFNτ gene using polymerase chain reaction. The linker at the 5' end allowed the placement of the synthetic IFNτ gene in correct reading with the ubiquitin coding sequence present in the yeast cloning vector pBS24Ub (Chiron Corp., Emeryville, CA). The linker also constructed a ubiquitin-IFNτ junction region that allowed *in vivo* cleavage of the ubiquitin sequences from the IFNτ sequences. The 5' oligonucleotide also encoded a *Sac*II restriction endonuclease cleavage site. The 3' oligonucleotide contained a *Stu*I cleavage site.

The vector carrying the synthetic IFNτ gene (Example 3) was isolated from *E. coli* strain "XLI-BLUE" by the alkaline lysis method. Isolated vector was diluted 500-fold in 10 mM Tris, pH 8.0/1 mM EDTA/10 mM NaCl. The PCR reaction was performed in a 100 µl volume using *Taq* DNA polymerase and primers SEQ ID NO:13/SEQ ID NO:14. The amplified fragments were digested with StuI and *Sac*II. These digested fragments were ligated into the *Sac*II and *Sma*I sites of "pBLUESCRIPT+(KS)."

The resulting plasmid was named pBSY-IFNτ. The DNA sequence was verified using double stranded DNA as the template.

### B. Construction of the Expression Plasmid.

Plasmid pBSY-IFNτ was digested with *Sac*II and *Eco*RV and the fragment containing the synthetic IFNτ gene was isolated. The yeast expression vector pBS24Ub (Sabin, *et al*.; Ecker, *et al*.) was digested with *Sal*I. Blunt ends were generated using T4 DNA polymerase. The vector DNA was extracted with phenol and ethanol precipitated (Sambrook, *et al.,* 1989). The recovered linearized plasmid was digested with *Sac*II, purified by agarose gel electrophoresis, and ligated to the *Sac*II-*Eco*RV fragment isolated from pBSY-IFNτ. The resulting recombinant plasmid was designated pBS24Ub-IFNτ.

The recombinant plasmid pBS24Ub-IFNτ was transformed into *E. coli.* Recombinant clones containing the IFNτ insert were isolated and identified by restriction enzyme analysis. Plasmid DNA from clones containing IFNτ coding sequences was used for transformation of *S. cerevisiae* (Rothstein, 1986). Transformation mixtures were plated on uracil omission medium and incubated for 3-5 days at 30°C. Colonies were then streaked and maintained on uracil and leucine omission medium (Rothstein, 1986).

### C. Expression Experiments.

For small-scale expression, a single colony of *S*. *cerevisiae* AB116 containing pBS24Ub-IFNτ was picked from a leucine and uracil omission plate and grown at 30°C in YEP medium (1% yeast extract, 2% peptone) containing 1% glucose for inducing conditions or 8% glucose for non-inducing conditions. Cell lysates were recovered and subjected to SDS-PAGE in 15% acrylamide, 0.4% bisacrylamide (Sambrook, *et al.,* 1989). The fractionated proteins were visualized by Coomassie blue staining.

Recombinant IFNτ was visualized specifically by immunoblotting with monoclonal antibody or polyclonal antiserum against ovine IFNτ upon electrotransfer of the fractionated cell extract to "NYTRAN" paper (Rothstein, 1986).

For large-scale expression, pBS24-IFNτ was grown for 24 hours at 30°C in 5 × uracil and leucine omission medium containing 8% glucose. This culture was then diluted 20-fold in YEP medium containing 1% glucose and further incubated for another 24-36 hours.

Cells were harvested by centrifugation, washed in 50 mM Tris, pH 7.6,/1 mM EDTA and resuspended in wash buffer containing 1 mM PMSF. The cells were lysed using a Bead-beater apparatus (Biospec Products, Bartlesville, OK). The lysate was spun at 43,000 × g for 20 minutes. The supernatant fraction was recovered and subjected to the purification protocol described below.

### D. Purification of r-IFNτ from Yeast Cell Lysate.

The supernatant was loaded on a 1 × 10 cm DEAE column and washed with 10 mM Tris, pH 8.0. Retained proteins were eluted with a 300 ml, 0 to 0.5 M NaCl gradient in 10 mM Tris, pH 8.0. Three-milliliter fractions were collected. Ten-microliter samples of fractions 17-26 containing the recombinant (r-IFNτ) were electrophorectically separated on 15% SDS-polyacrylamide gels. The gels were stained with Coomassie blue.

Fractions 18, 19, and 20 contained largest amount of r-IFNτ. These fractions were loaded individually on a 1.5 × 90 cm Sephadex S-200 column and proteins were resolved in two peaks. Aliquots of each protein peak (25 µl) were electrophoretically separated on 15% SDS-polyacrylamide gels and the proteins visualized with Coomassie staining.

Purified r-IFNτ-containing fractions were combined and the amount of r-IFNτ quantified by radioimmunoassay (Vallet, *et al*., 1988). Total protein concentration was determined by using the Lowry protein assay (Lowry, *et al.*, 1951).

Microsequencing of purified r-IFNτ demonstrated identity with native IFNτ through the first 15 amino acids, confirming that the ubiquitin/r-IFNτ fusion protein was correctly processed *in vivo*.

Purified r-IFNτ exhibited 2 to 3 × 10⁸ units of antiviral activity per milligram of protein (*n* = 3 replicate plates) which is similar to the antiviral activity of IFNτ purified from conceptus-conditioned culture medium (2 × 10⁸ U/mg).

### EXAMPLE 5

### Southern Blot Analysis of Human High Molecular Weight DNA

Human venous blood samples from healthy donors were collected in heparinized tubes and peripheral blood lymphocytes were isolated by density-gradient centrifugation using a Ficoll-Isopaque gradient (1.077 g/ml) (Sigma Chemical Co.). High molecular weight (HMW) DNA was isolated from these cells (Sambrook, *et al.,* 1989).

Two 10 µg samples of HMW DNA were digested with the restriction endonucleases *Hind*III or *Pst*I (Promega) for 2 hours at 37°C, and the DNA fragments electrophoretically separated in a 0.8% agarose gel (Bio-Rad, Richmond, CA) at 75 volts for 8 hours. The DNA fragments were transferred onto a nylon membrane (IBI-International Biotechnologies, Inc., New Haven, CT). The membrane was baked at 80°C for 2 hours and incubated at 42°C for 4 hours in the following prehybridization solution: 5 × SSC (1 × SSC is 0.15 M NaCl and 0.15 M sodium citrate), 50% vol/vol formamide, 0.6% (wt/vol) SDS, 0.5% (wt/vol) nonfat dry milk, 20 mM Tris-HCl (pH 7.5), 4 mM EDTA, and 0.5 mg/ml single stranded herring sperm DNA (Promega).

The filter was then incubated in a hybridization solution (5 × SSC, 20% vol/vol formamide, 0.6% (wt/vol) SDS, 0.5% (wt/vol) nonfat dry milk, 20 mM Tris-HCl (pH 7.5), 4 mM EDTA, and 2 × 10⁸ cpm/ml ³²P-labelled OvIFNτ cDNA (Imakawa, *et al*., 1987)) for 18 hours at 42°C. The filter was washed at 42°C for 15 minutes with 2 × SSC and 0.1% (wt/vol) SDS and exposed to X-ray film (XAR, Eastman Kodak, Rochester, NY) at -80°C for 48 hours in the presence of an intensifying screen.

Autoradiography detected a hybridization signal at approximately 3.4 kb in DNA digested with PstI and a slightly smaller (≈ 3.0 kb) fragment in the *Hind*III digested DNA. These results indicate the presence of human DNA sequences complementary to the OvIFNτ cDNA probe.

### EXAMPLE 6

### Isolation of Partial Sequence of Human IFN cDNA by PCR

Two synthetic oligonucleotides (each 25-mer), corresponding to the sequence 231 to 255 (contained in SEQ ID NO:13) and 566 to 590 (contained in SEQ ID NO:14) of OvIFNτ cDNA (numbering relative to the cap site, Imakawa, *et al.,* 1987) were synthesized. These primers contained, respectively, cleavage sites for the restriction endonucleases *PstI* and *Eco*RI. SEQ ID NO:13 was modified to contain the *EcoRI* site, which begins at position 569.

DNA was isolated from approximately 1 × 10⁵ plaque forming units (pfu) of the following two cDNA libraries: human term placenta (Clontech, Inc., Palo Alto, CA) and human term cytotrophoblast (Dr. J.F. Strauss, University of Pennsylvania, Philadelphia PA). The DNA was employed in polymerase chain reaction (PCR) amplifications (Mullis; Mullis, *et al.;* Pekin Elmer Cetus Corp. Norwalk CT). Amplification reactions were carried out for 30 cycles (45°C, 1m; 72°C, 2m; 94°C, lm) (thermal cycler and reagents, Perkin Elmer Cetus) using primers SEQ ID NO:13/SEQ ID NO:14.

Amplification products were electrophoretically separated (100 volts in a 1.5% agarose gel (Bio-Rad)) and transferred onto a nylon membrane (IBI). The membrane was baked at 80°C for 2 hours and prehybridized and hybridized with ³²P-labelled OvIFNτ cDNA as described above. The membrane was washed in 5 × SSC/0.1% (wt/vol) SDS for 5 minutes at 42°C and in 2 × SSC/0.1% (wt/vol) SDS for 2 minutes at 42°C. It was then exposed at -80°C to "XAR" (Eastman Kodak) X-ray film for 24 hours in the presence of an intensifying screen. An amplification product that hybridized with the labelled probe DNA was detected.

PCR was performed again as directed above. Amplified products were digested with the restriction endonucleases *Eco*RI and *PstI* (Promega) for 90 minutes at 37°C. The resulting DNA fragments were electrophoretically separated as described above and the band containing the IFNτ amplification product was excised from the gel. DNA fragments were recovered by electroelution, subcloned into *Eco*RI/*Pst*I digested-dephosphorylated plasmid pUC19 and transformed into *E. coli* strain JM101 (Promega) by calcium chloride method (Sambrook, *et al.,* 1989). The plasmids were isolated and the inserted amplification product sequenced using the dideoxy termination method (Sanger, *et al.,* 1977; "SEQUENASE" reactions, United States Biochemical, Cleveland, OH). Nucleotide sequences were determined, and comparison of these as well as the deduced amino acid sequences to other IFN sequences were performed using DNA Star Software (Madison, WI).

Comparison of the sequences of these clones revealed three different clones: from the human placental library, Clone 15 (306 bp) and Clone 21 (315 bp), which exhibit 95% identity in their nucleotide sequences; from the cytotrophoblast library clone CTB 35 (301 basepairs), which shares 95% and 98% identity with Clone 15 and Clone 21, respectively.

### EXAMPLE 7

### Isolation of Full-Length Human IFNτ Gene

Ten micrograms PBMC HMW DNA was digested with restriction endonuclease *Eco*RI and subjected to electrophoretic analysis in a 0.8% agarose gel. A series of samples containing ranges of DNA fragments sized 1.5 to 10 kb *(e.g.,* 1.5 to 2.5 kb, 2.5 kb to 3 kb) were excised from the gel. The DNAs were electroeluted and purified. Each DNA sample was amplified as described above using the OvIFNτ primers. The DNA molecules of any sample that yielded a positive PCR signal were cloned into λgt11 (the subgenomic λgt11 library).

### A. PCR Identification of Clones Containing Sequences Complementary to OvIFNτ.

The λgt11 phage were then plated for plaques and plaque-lift hybridization performed using the ³²P-labelled OvIFNτ cDNA probe. Approximately 20 clones were identified that hybridized to the probe.

Plaques that hybridized to the probe were further analyzed by PCR using the OvIFNτ primers described above. Six plaques which generated positive PCR signals were purified. The phage DNA from these clones was isolated and digested with *Eco*RI restriction endonuclease. The DNA inserts were subcloned into pUC19 vectors and their nucleotide sequences determined by dideoxy nucleotide sequencings.

### B. Hybridization Identification of Clones Containing Sequences Complementary to PCR-Positive Phage.

Recombinant phage from the λgt11 subgenomic library were propagated in *E. coli* Y1080 and plated with *E. coli* Y1090 at a density of about 20,000 plaques/150 mm plate. The plates were overlaid with duplicate nitrocellulose filters, which were hybridized with a ³²P-labelled probe from one of the six human IFNτ cDNA clones isolated above. Three clones giving positive hybridization signals were further screened and purified. The phage DNAs were isolated, digested with *Eco*RI, subcloned into pUC19 vector and sequenced. The three clones yielded sequence information for over 800 bases relative to cap site (clones were sequenced in both orientations). The nucleic acid sequence information is presented as SEQ ID NO:11 and the predicted protein coding sequence is presented as SEQ ID NO:12. Comparison of the predicted mature protein sequence (SEQ ID NO:12) of this gene to the predicted protein sequence of OvIFNτ is shown in Figure 3.

### EXAMPLE 8

### Analysis of the Presence of HuIFNτ mRNA by RT-PCR

Human placental cDNA libraries and an ovine cDNA library, constructed from day 15-16 conceptuses, were analyzed by hybridization to the OvIFNτ cDNA probe, described above. cDNAs were size-fractionated on agarose gels and transferred to filters (Maniatis, *et al*.; Sambrook, *et al*.). Southern blot analysis with OvIFNτ probe showed that the autoradiographic signals from human cDNA libraries were approximately 1/100 of the signal obtained using the OvIFNτ cDNA library.

The presence of HuIFNτ mRNA in human term placenta and amniocytes (26 weeks, 2 million cells) was analyzed by using reverse transcriptase-PCR (RT-PCR) method (Clontech Laboratories, Palo Alto CA).

Total cellular RNA (tcRNA) isolated from human placenta, amniocytes and ovine conceptuses were reverse transcribed using the primer SEQ ID NO:14. The primer SEQ ID NO:13 was then added to the reaction and polymerase chain reaction carried out for 40 cycles. The PCR products were size fractionated on agarose gels and transferred to filters. The DNA on the filters was hybridized with ³²P-labelled OvIFNτ and HuIFNτ cDNAs. The results of these analyses demonstrate the presence of human IFNτ mRNA in the feto-placental annex. The aminocytes also expressed the messages corresponding to OvIFNτ primers and human probe.

In addition, a RT-PCR analysis for the presence of HuIFNτ was applied to the tcRNA isolated from human adult lymphocytes. A densitometric analysis revealed that IFNτ mRNA exists in lymphocytes.

### EXAMPLE 9

### In Situ Hybridization

### A. Tissue

Slides of semiserial 5-µ paraffin embedded sections from four healthy, different term and first trimester human placentas were examined.

### B. cRNA Probe Preparation

From the cDNA clone isolated from OvIFNτ amplified library a fragment corresponding to the OvIFNτ cDNA bases #77-736 (base #1 is cap site; open reading frame of OvIFNτ cDNA is base #81-665; Figure 7) was subcloned into the transcription vector, pBS (New England Biolabs). Several pBS clones were isolated, subcloned, and their nucleotides sequenced. From this clone a 3' fragment (bases #425-736) was excised using the restriction endonucleases *Nla*IV and *Eco*RI and subcloned into the transcription vector pBS. This vector was designated pBS/OvIFNτ.

After linearization of the pBS/OvIFNτ plasmid, an antisense cRNA probe was synthesized by in *vitro* transcription (Sambrook, *et al.,* 1989) using T₇ RNA polymerase (Stratagene). A trace amount of ³H-CTP (NEN-DuPont, Cambridge, MA) was used in the transcription reaction. dUTP labeled with digoxigenin (Boehringer-Mannheim, Indianapolis, IN) was incorporated into the cRNA and yield was estimated through TCA precipitation and scintillation counting.

### C. Hybridization

*In situ* hybridization was performed using the anti-sense RNA probe, as described by Lawrence, *et al*. (1985) with the following modifications. Deparaffinized and hydrated sections were prehybridized for 10 minutes at room temperature in phosphate buffered saline (PBS) containing 5 mM MgCl₂. Nucleic acids in the sections were denatured for 10 minutes at 65°C in 50% formamide/2 × SSC. Sections were incubated overnight at 37°C with a hybridization cocktail (30 µl/slide) containing 0.3 µg/ml digoxigenin-labelled cRNA probe and then washed for 30 minutes each at 37°C in 50 formamide/1 × SSC. Final washes were performed for 30 minutes each at room temperature in 1 × SSC and 0.1 × SSC. The sections were blocked for 30 minutes with 0.5% Triton X-100 (Sigma) and 0.5% non-fat dry milk.

Hybridization signal was detected using purified sheep antidioxigenin Fab fragments conjugated to alkaline phosphatase (Boehringer-Mannheim). After unbound antibody was removed, nitroblue tetrazolium/5-bromo-4-chloro-3-indolyl-phosphate substrate (Promega) and levamisole (Bector Laboratories, Burlingame, CA) were added for signal detection via colorimetric substrate generation. The tissues were counterstained in methyl green (Sigma), dehydrated, and mounted.

As a control, some tissue sections were pretreated with 100 µg/ml of pancreatic RNaseA (Sigma) for 30 minutes at 37°C. The RNase was inactivated on the slide with 400 units of RNase inhibitor (Promega). The slides were then washed twice in 250 ml of PBS/5 mM MgCl₂. In other control experiments, tRNA (Sigma) was substituted for the digoxigenin probes.

Specific hybridization was observed in all term and first trimester placental tissues in three separate experiments with various OvIFNτ cRNA probe concentrations and blocking reagents.

First trimester placental villi composed of an outer layer of syncytiotrophoblast, an underlying layer of cytotrophoblast, and a central stromal region with various types of mesenchymal cells, displayed the highest transcript level of IFNτ in the cytotrophoblast cells. Less intense but detectable levels were present in both the syncytiotrophoblast and stromal cells. A similar pattern of transcript expression was demonstrated in the placental villi of term tissue but the level of signal detection was low. First trimester extravillous trophoblast displayed the highest amount of message and stained positive when present in the maternal blood spaces.

### EXAMPLE 10

### Antiviral Activity of IFNτ

The relative specific activity of OvIFNτ, purified to homogeneity, was evaluated in antiviral assays. The antiviral assays were performed essentially as described above in Example 2. Specific activities are expressed in antiviral units/mg protein obtained from antiviral assays using either Madin-Darby bovine kidney (MDBK) cells or sheep normal fibroblasts (Shnf). All samples were assayed simultaneously to eliminate interassay variability. The results, presented in Table 3, are the means of four determinations where the standard deviation was less than 10% of the mean.

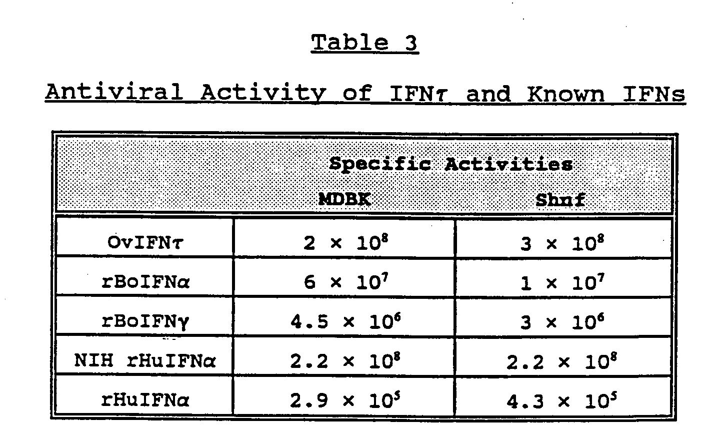

IFNτ had a higher specific activity than either rBoIFNα or rBoIFNγ (Table 3). The NIH standard preparation of rHuIFNα had a similar specific activity, while a commercial preparation of rHuIFNα exhibited low specific antiviral activity. Comparable relative antiviral activity was demonstrated using either bovine or ovine cells.

### EXAMPLE 11

### Anti-Retroviral Activity and Cytotoxic Effects of IFNτ

Highly purified OvIFNτ was tested for anti-retroviral and cytotoxic effects on feline peripheral blood lymphocytes exposed to the feline immunodeficiency retrovirus. This lentivirus produces a chronic AIDS-like syndrome in cats and is a model for human AIDS (Pederson, *et al.,* 1987). Replication of the virus in peripheral blood lymphocytes is monitored by reverse transcriptase activity in culture supernatants over time. The data from these assays are presented in Table 4.

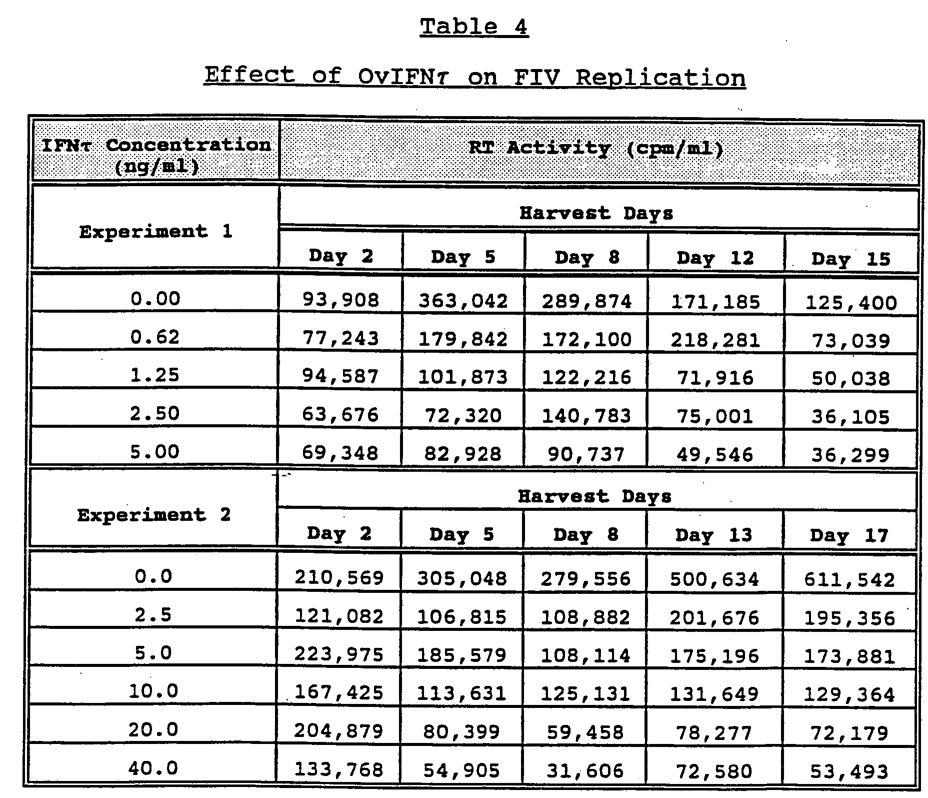

Addition of OvIFNτ produced a rapid, dose-dependent decrease in reverse transcriptase (RT) activity (Table 4). While concentrations as low as 0.62 ng/ml of IFNτ inhibited viral replication, much higher concentrations (40 ng/ml) having greater effects on RT-activity were without toxic effects on the cells. The results suggest that replication of the feline immunodeficiency virus was reduced significantly compared to control values when cells were cultured in the presence of OvIFNτ.

IFNτ appeared to exert no cytotoxic effect on the cells hosting the retrovirus. This was true even when IFNτ was present at 40 ng per ml of culture medium.

### EXAMPLE 12

### Effects of IFNτ on HIV Infected Human Peripheral Lymphocytes

IFNτ was also tested for activity against HIV infection in human cells. Human peripheral blood lymphocytes, which had been infected with HIV (Crowe, *et al*.), were treated with varying concentrations of OvIFNτ. Replication of HIV in peripheral blood lymphocytes was monitored by reverse transcriptase activity in culture supernatants over time. Reverse transcriptase activity was measured essentially by the method of Hoffman, *et al*. The data from these assays are presented in Table 5.

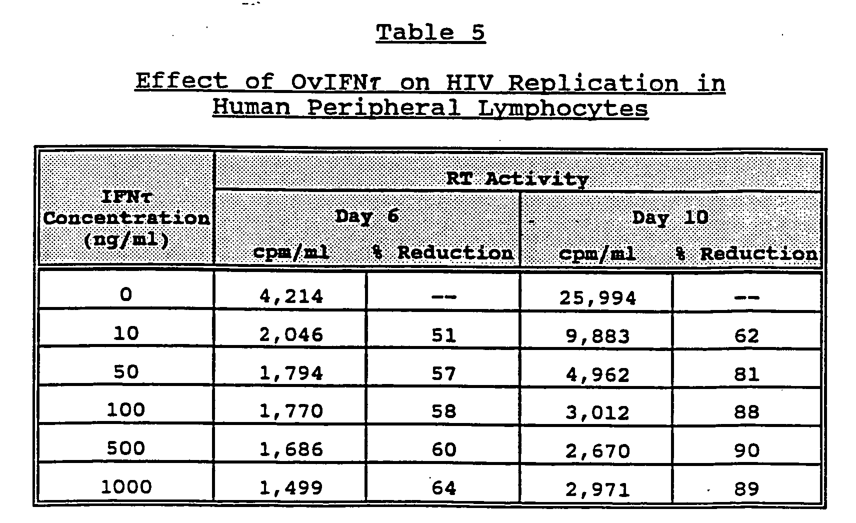

As shown in Table 5, concentrations of OvIFNτ produced significant antiviral effects. A concentration of only 10 ng/ml resulted in over a 50% reduction in RT activity after only six days. A concentration of 500 ng/ml resulted in a 90% reduction in RT activity within 10 days.

The viability of human peripheral blood lymphocytes after treatment with IFNτ, over a range of concentrations for 3-13 days, was evaluated by trypan blue exclusion. The results of this viability analysis are presented in Table 6.

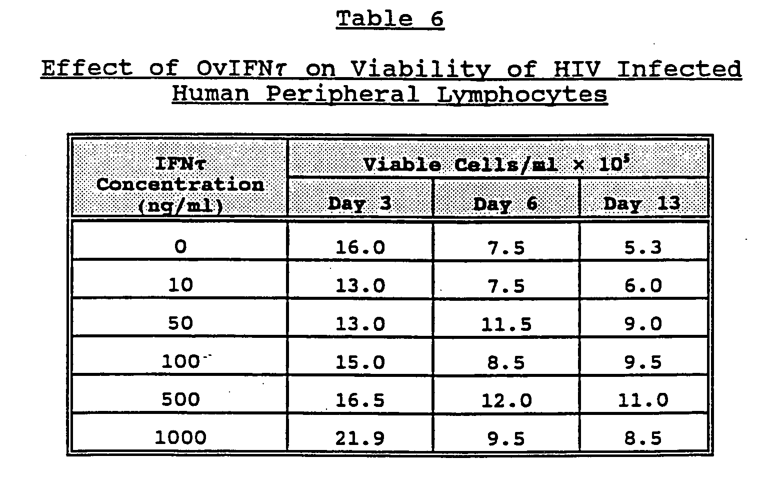

The data presented in Table 6 show no evidence of cytotoxic effects attributable to the administration of IFNτ.

### EXAMPLE 13

### Inhibition of Cellular Growth

The effects of IFNτ on cellular growth were also examined. Anti-cellular growth activity was examined using a colony inhibition assay. Human amnion (WISH) or MDBK cells were plated at low cell densities to form colonies originating from single cells. Cells were cultured at 200 or 400 cells/well in 24 well plates in HMEM supplemented with 2% fetal bovine serum (FBS) and essential and nonessential amino acids. Various dilutions of interferons were added to triplicate wells, and the plates were incubated for 8 days to allow colony formation. Colonies were visualized after staining with crystal violet, and counted. Cell cycle analysis was performed with HMEM containing 0.5% "spent" media for an additional 7 days. WISH cells were used without being synchronized.

For examination of IFNτ activity, cells were replated at 2.5 × 10⁵ cells/well in HMEM with 10% FBS in 6 well plates. Various dilutions of IFNτ alone or in combination with peptides were added to achieve a final volume of 1 ml. Plates were incubated at 37°C in 5% Co₂ for 12, 15, 18, 24, or 48 hours. Cells were treated with trypsin, collected by low speed centrifugation and washed. The cell pellet was blotted dry and 250 µl of nuclear staining solution (5 mg propidium iodide, 0.3 ml NP40 and 0.1 gm sodium citrate i 100 ml distilled H₂O) was added to each tube. The tubes were incubated at room temperature. After 10 minutes, 250 µl of RNase (500 units/ml in 1.12% sodium citrate) was added per tube and incubated an additional 20 minutes. Nuclei were filtered through 44 µm mesh, and analyzed on a FACStar (Becton Dickinson, Mountain View, CA) using the DNA Star 2.0 software.

In the cellular growth assay using colony formation of both the bovine epithelial line, MDBK, and the human amniotic line, WISH, OvIFNτ inhibited both colony size and number. Ovine IFNτ was more effective than human IFNα on the human cell line; thus, it is very potent in cross-species activity. Its activity was dose-dependent, and inhibition of proliferation could be observed at concentrations as low as 1 unit/ml. Concentrations as high as 50,000 units/ml (units of antiviral activity/ml) stopped proliferation, while cell viability was not impaired.

Cell cycle analysis by flow cytometry with propidium iodide-stained WISH cells revealed an increased proportion of cells in G2/M after 48 hours of OvIFNτ treatment. IFNτ, therefore, appears to inhibit progress of cells through S phase. Ovine IFNτ antiproliferative effects can be observed as early as 12 hours after the initiation of culture and are maintained through 6 days.

The results presented above demonstrate both the antiproliferative effect of IFNτ as well as its low cytotoxicity.

### EXAMPLE 14

### Further Antiproliferative Effects of IFNτ

The antiproliferative effects of OvIFNτ were studied for a rat cell line and a bovine cell line. The rate of ³H-thymidine incorporation was used to assess the rate of cellular proliferation.

Rat (MtBr7 .c5) or bovine kidney (MDBK) cells were seeded in phenol red-free DME-F12 medium supplemented with 3% dextran-coated charcoal stripped Controlled Process Serum Replacement 2 (CPSR 2, Sigma) and 5% dextran-coated charcoal stripped fetaL bovine serum (FBS). After attaching for approximately 15-18 hours, the cells were washed once with serum-free DME-F12 medium. The medium was replaced with phenol red-free DME-F12 medium supplemented with 3% stripped CPSR2, 1% stripped FBS ("3/1" medium) or 3/1 medium containing OvIFNτ at various units of antiviral activity as determined in the vesicular stomatitis virus challenge assay for interferons (Example 2). Media containing a similar dilution of buffer (undiluted buffer = 10 mM Tris, 330 mM NaCl, [TS]), in which the OvIFNτ was dissolved was used for controls.

Cells were pulse labeled with ³H-thymidine for 2 hours at approximately 48 hours post-treatment. The trichloroacetic acid (TCA) precipitable incorporated counts were determined by scintillation counting. Three replicates were included per treatment. Mean values for OvIFNτ treatments were compared to samples containing comparable dilutions of carrier TS buffer. Results of these experiments are shown in Table 7.

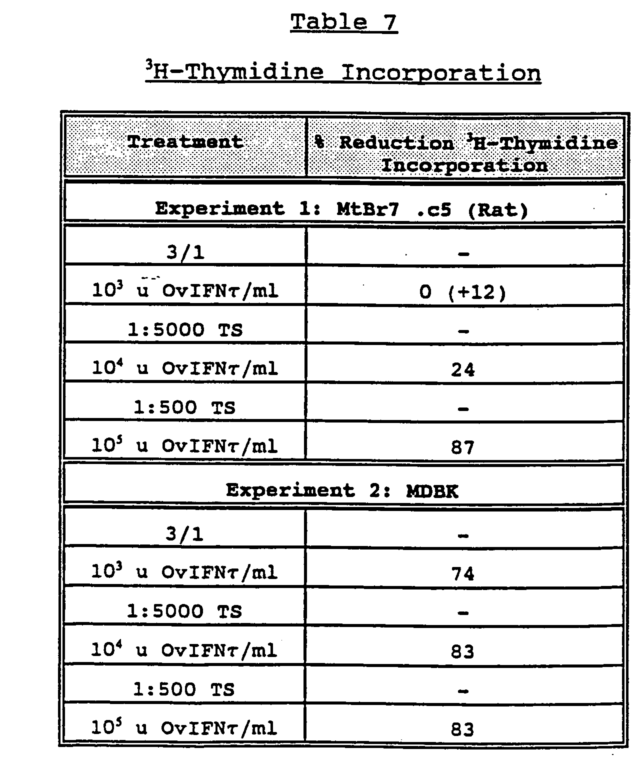

As can be seen from Table 7, OvIFNτ drastically reduced the rate of cellular proliferation (based on thymidine incorporation) for each of the cell lines tested.

### EXAMPLE 15

### Antiproliferative Effects of IFNτ on Human Tumor Cell Lines

The antiproliferative activity of OvIFNτ on human tumor cell lines was evaluated by measuring the rate of ³H-thymidine incorporation into cells which have been treated with OvIFNτ.

For experiments on tumor lines that grow in suspension, 1 ml of cells were plated at from 2.5 - 5 X 10⁵ cells/well in 24-well plates. Triplicate wells received either the appropriate media, 100, 1,000 or 10,000 units/ml of OvIFNτ or equivalent antiviral concentrations of rHuIFNα2A (Lee Biomolecular). After 48 hours of incubation, cells were counted and viability assessed by trypan blue exclusion.

Adherent tumor lines were plated at 2.5 X 10⁵ cells/well in 1 ml in 6-well plates. They received interferon treatments as just described, but were trypsinized prior to counting.

Significant differences between treatments were assessed by an analysis of variance followed by Scheffe's F-test. Cell cycle analysis was performed by flow cytometry using propidium iodide.

### A. Breast Adenocarcinoma Cells.

Human MCF7 breast adenocarcinoma cells were seeded from logarithmically growing cultures in phenol red-free DME-F12 medium supplemented with 3% dextran-coated charcoal stripped CPSR and 5% dextran-coated FBS. After attaching for approximately 15-18 hours, the cells were washed once with serum-free DME-F12 medium. The medium was replaced with phenol red-free DME-F12 medium supplemented with 3% stripped CPSR2, 1% stripped FBS ("3/1" medium) or 3/1 medium containing OvIFNτ at the indicated number of units of antiviral activity as determined in the vesicular stomatitis virus challenge assay for interferons. Media containing a similar dilution of buffer (undiluted buffer = 10 mM Tris, 330 mM NaCl [TS]) was used for controls. Cells were pulse labeled with ³H-thymidine for 2 hours at approximately 48 hours post-treatment.

The trichloroacetic acid (TCA) precipitable incorporated counts were determined by scintillation counting. Three replicates were included per treatment. Mean values for OvIFNτ treatments were compared to samples containing comparable dilutions of carrier TS buffer. The results of these analyses are shown in Table 8.

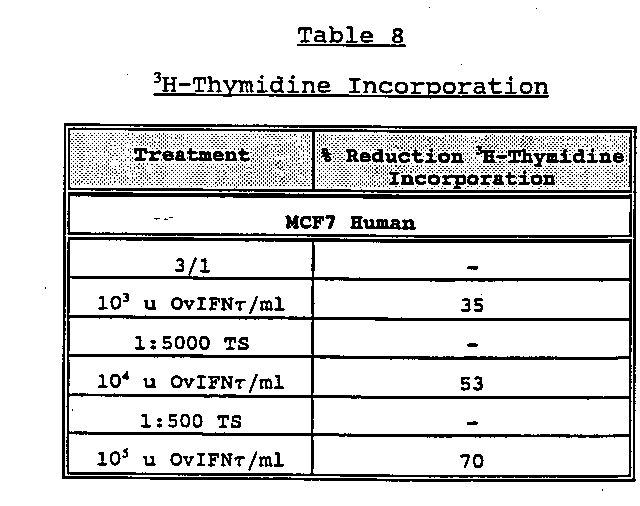

As can be seen from the results shown in Table 8, OvIFNτ was able to substantially reduce the rate of ³H-thymidine incorporation in the human carcinoma cell line. This demonstrates the efficacy of OvIFNτ in inhibiting tumor cell proliferation, in particular, mammary tumor cell proliferation.

### B. Human Promyelocytic Leukemia.

A comparison of the antiproliferative effects of OvIFNτ and IFNα was conducted using HL-60 (human leukemia) cells (Foa, *et al*.; Todd, *et al.)* essentially as described above for MDBK cells. Both OvIFNτ and rHuIFNα inhibit HL-60 cell proliferation. Results of one of three replicate experiments are presented as mean % growth reduction ± SD in Figure 4. Figure 4 shows that both OvIFNτ and IFNα were able to drastically reduce growth of HL-60 cells. The growth reduction for each compound exceeded 60% for each concentration tested. At 10,000 units/ml, OvIFNτ caused an approximately 80% reduction in growth while IFNα caused a 100% reduction in growth.

However, the data presented in Figure 4 reveal, that a substantial factor in the ability of IFNα to reduce growth was its toxic effect on the cells. At 10,000 units/ml, the toxicity of IFNα resulted in less than 25% of the cells remaining viable. By contrast, nearly 100% of the cells remained viable when OvIFNτ was applied at 10,000 units/ml.

Figure 5 presents data demonstrating that rHuIFNα is cytotoxic. In the figure, results of one of three replicate experiments are presented as mean % viability ± SD.

### C. Human Cutaneous T Cell Lymphoma.

The cutaneous T cell lymphoma, HUT 78, responded similarly to HL-60 when treated with IFNτ (Figure 9). Both OvIFNτ and rHuIFNα reduce HUT 78 cell growth, but 10,000 units/ml of rHuIFNα decreased the cell number below that originally plated (5X10⁵). This is indicative of a reduction in cell viability to approximately 60%.

Cell cycle analysis (performed by cell flow cytometry) revealed an increased proportion of cells in G2/M phase of the cell cycle upon 48 hours of treatment with both interferons (Table 10). In Table 10 the results from one of three replicate experiments are presented as the percentage of cells in each phase of the cell cycle. 10,000 events were analyzed per sample.

This result is likely due to the slower progress of cells through the cell cycle. In the sample treated with 10,000 units/ml of rHuIFNα, a large percentage of events with low forward and high side scatter, identifying dead cells, were present. This is consistent with the data obtained from proliferation experiments, where only OvIFNτ inhibited HUT 78 proliferation without toxicity.

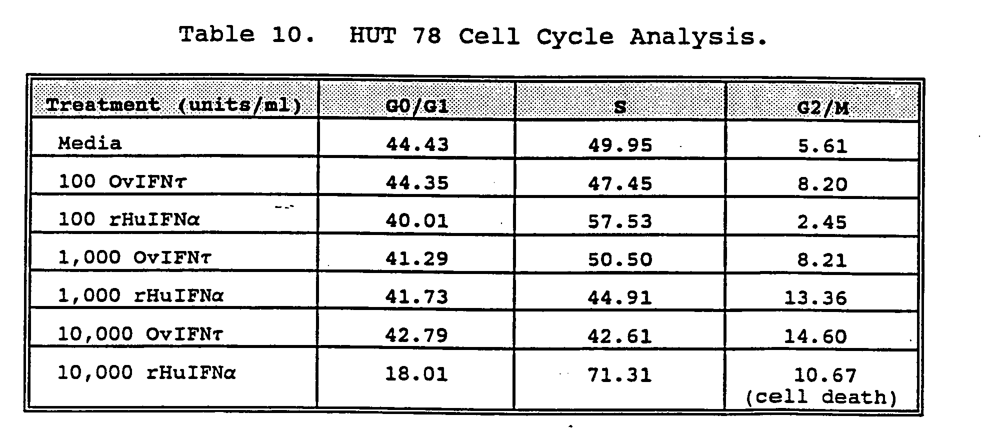

### D. Human T Cell Lymphoma.

The T cell lymphoma cell line H9 was slightly less sensitive to the antiproliferative effects of the IFNs than the tumor cell lines described above. Results of one of three replicate experiments are presented in Figure 10 as mean % growth reduction ± SD. While rHuIFNα was not toxic to the H9 cells, it failed to inhibit cell division significantly at any of the concentrations examined. In contrast, OvIFNτ was observed to reduce H9 growth by approximately 60% (Figure 10). Thus, only OvIFNτ is an effective growth inhibitor of this T cell lymphoma.

The results presented above demonstrate both the antiproliferative effect of IFNτ as well as its low cytotoxicity.

### EXAMPLE 16

### Preliminary In Vivo Treatment with OvIFNτ

Three groups of 4 C57Bl/6 mice per group were given 2.5 X 10⁴ B16-F10 cells via the tail vein: B16-F10 is a syngeneic mouse transplantable tumor selected because of its high incidence of pulmonary metastases (Poste, *et al*., 1981). Interferon treatment was initiated 3 days after the introduction of the tumor cells. Each mouse received 100 µl of either PBS alone, PBS containing 1 X 10⁵ units of OvIFNτ, or PBS containing 1 X 10⁵ units of recombinant murine IFNα (MuIFNα), i.v. per day for 3 consecutive days.

Mice were sacrificed at 21 days and the lungs were preserved in 10% buffered formalin. The frequency of pulmonary metastases were compared between control mice (PBS), OvIFNτ-treated mice, and MulFNα-treated mice. The results of these *in vivo* administrations demonstrated that OvIFNτ dramatically reduced B16-F10 pulmonary tumors. These results support the use of IFNτ as an efficacious antineoplastic agent *in vivo.*

### EXAMPLE 17

### Competitive Binding of IFNτ Peptide Fragments

### A. The Ability of IFNτ-Based Peptides to Block IFNτ and IFN-α Antiviral Activity.

Overlapping synthetic peptides were synthesized corresponding to the entire IFNτ sequence (Figure 6). Average hydropathicity values were calculated by taking the sum of the hydropathy values for each amino acid divided by the total number of amino acids in each sequence. Hydropathy values were taken from Kyte, *et al*. (1982).

These peptides were of approximately the same molecular weight but differed slightly in overall hydrophilicity. Despite this difference, all peptides were antigenic as demonstrated by the production of rabbit antisera with titers greater than 1:3,000 as assessed by ELISA (Harlow, *et al*.).

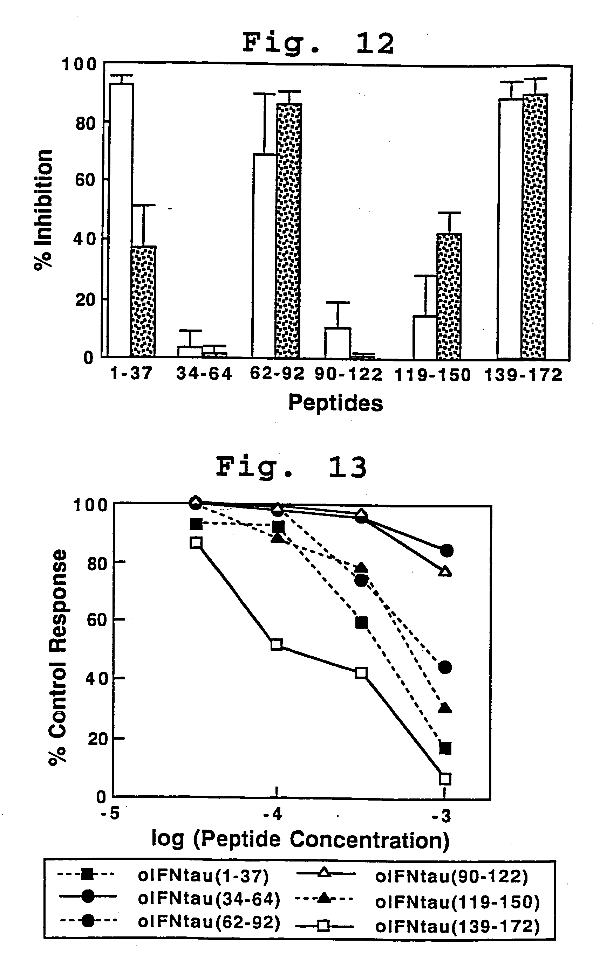

The peptides were used to inhibit the antiviral activity (Example 2) of OvIFNτ and rBoIFNα. The results of this analysis are presented in Figure 12: 1 mM N- and C-terminal peptides both effectively blocked the antiviral activity of OvIFNτ using MDBK cells. A third peptide, representing amino acids 62-92, also reduced IFNτ antiviral activity (70% inhibition). The peptide OvIFNτ (119-150) showed minimal inhibitory activity. The OvIFNτ (34-64) and (90-122) peptides had no apparent inhibitory activity.

Peptide inhibition of OvIFNτ antiviral activity was also examined as follows. Monolayers of Madin Darby bovine kidney cells were incubated with 40 units/ml OvIFNτ in the presence or absence of various concentrations of OvIFNτ peptides (see Figure 13). Results in Figure 13 are expressed as the percent of control antiviral activity: that is, in the absence of any competing peptide. Data presented are the means of 6 replicate experiments. The data demonstrate that inhibition by OvIFNτ (1-37), (62-92), (119-150), and (139-172) were significantly different than OvIFNτ (34-64) and (90-122) at 10⁻³ M and 3 x 10⁻³ M. OvIFNτ (139-172) was significantly different than all other peptides at 10⁻³ M. Significance was assessed by analysis of variance followed by Scheffe's F test at p < 0.05. Thus, OvIFNτ (1-37) (62-92), (119-150), and (139-172), in particular (139-172), may represent receptor binding regions for IFNτ.

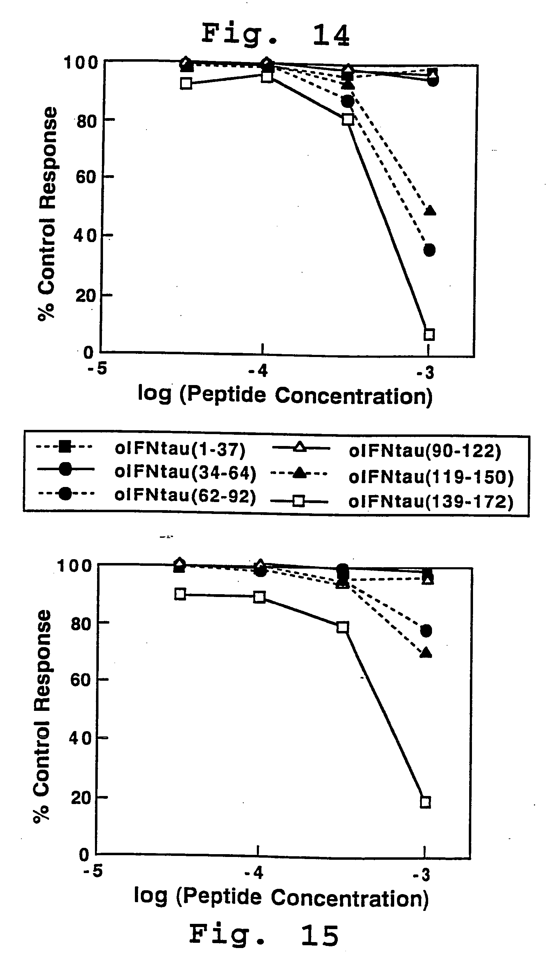

The ability of the OvIFNτ peptides to inhibit bovine IFNα (BoIFNα) antiviral activity was examined as follows. Monolayers of Madin Darby bovine kidney cells were incubated with 40 units/ml bovine IFNα in the presence or absence of various concentrations of OvIFNτ peptides. The results are presented in Figure 14 and are expressed as the percent of control antiviral activity in the absence of OvIFNτ peptides. The data presented are the means of 4 replicate experiments. The results indicate that inhibition by OvIFNτ (62-92), (119-150), and (139-172) were significantly different from OvIFNτ (1-37), (34-64) and (90-122) at 10⁻³ M. OvIFNτ (139-172) was significantly different than OvIFNτ (1-37), (34-64) and (90-122) at 3 x 10⁻³ M. Significance was assessed by analysis of variance followed by Scheffe's F test at p < 0.05. Thus, OvIFNτ (62-92), (119-150), and (139-172), in particular (139-172), may represent common receptor binding regions for IFNτ and bovine IFNα.

Peptide inhibition by OvIFNτ peptides of human IFNα antiviral activity was also examined. Monolayers of Madin Darby bovine kidney cells were incubated with 40 units/ml human IFNα in the presence or absence of various concentrations of OvIFNτ peptides. The results are expressed as the percent of control antiviral activity in the absence of OvIFNτ peptides. The data are presented in Figure 15 and are the means of 3 replicate experiments. OvIFNτ (139-172) was significantly different from all other peptides at 10⁻³ M. Significance was assessed by analysis of variance followed by Scheffe's F test at p < 0.05. Thus, OvIFNτ (139-172) may represent a common receptor binding region for IFNτ and various IFNα(s).

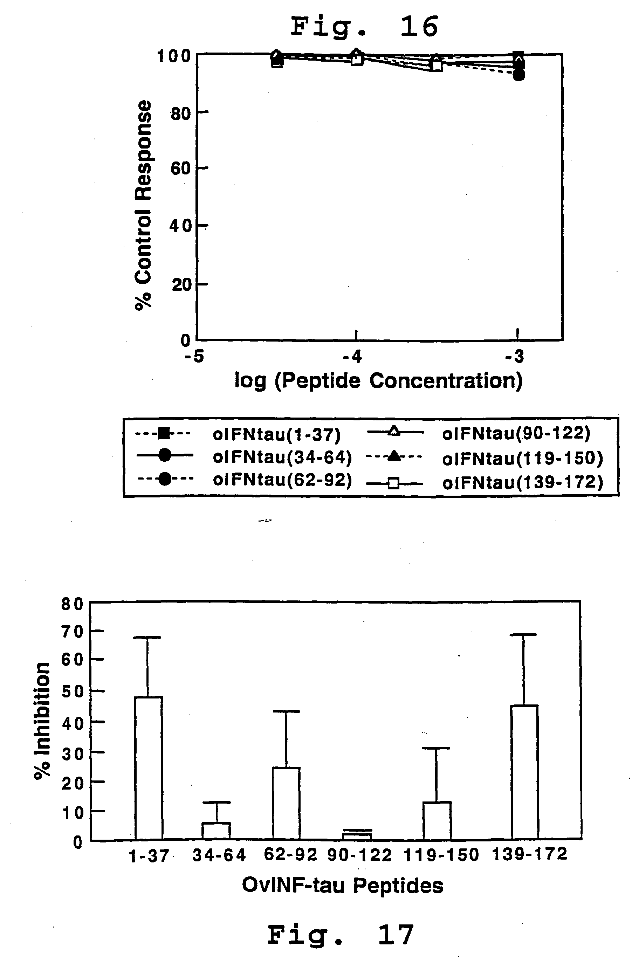

The OvIFNτ peptides described above appear to have no effect on the antiviral activity of IFNγ. Peptide inhibition of bovine IFNγ antiviral activity was evaluated as follows. Monolayers of Madin Darby bovine kidney cells were incubated with 40 units/ml bovine IFN gamma in the presence or absence of various concentrations of OvIFNτ peptides. Results are expressed as the percent of control antiviral activity in the absence of OvIFNτ peptides. The data are presented in Figure 16 and are the means of 3 replicate experiments. There were no significant differences among peptides as assessed by analysis of variance followed by Scheffe's F test at p < 0.05.

The two synthetic peptides OvIFNτ(1-37) and OvIFNτ(139-172) also blocked OvIFNτ anti-FIV and anti-HIV activity. Reverse transcriptase (RT) activity (Examples 12 and 13) was monitored over a 14 day period in FIV-infected FET-1 cells (1 X 10⁶/ml) and HIV-infected HPBL (1 X 10⁶/ml). Control cultures received no OvIFNτ. OvIFNτ was used at 100 ng/ml, and peptides were used at 200 µM. Data from a representative experiment are expressed as cpm/ml culture supernatant and are presented for FIV infected cells, Figure 11A, and HIV infected cells, Figure 11B. Both the N- and C-terminus of OvIFNτ appear to be involved in its anti-retroviral activity. While both peptides blocked FIV RT activity, only the C-terminal peptide, OvIFNτ(139-172), was an efficient inhibitor of vesicular stomatitis virus activity on the feline cell line, Fc9. Thus the C-terminal regions of type I IFNs may bind to common site on the type I IFN receptor, while the N-terminal region may be involved in the elicitation of unique functions.

### B. Anti-Peptide Sera.

The ability of anti-peptide antisera to inhibit OvIFNτ antiviral activity was also determined. Antipeptide antisera inhibition of OvIFNτ antiviral activity was evaluated as follows. Monolayers of MDBK cells were incubated with 20 units/ml of OvIFNτ in the presence a 1:30 dilution of either preimmune sera or antisera to each of the OvIFNτ peptides described above. In Figure 17 the data from duplicate experiments are presented as the mean percent inhibition of OvIFNτ antiviral activity produced by antipeptide antisera relative to the appropriate preimmune sera ± standard error. Significant differences were assessed by analysis of variance followed by Scheffe's F test at p < 0.05. Consistent with peptide inhibition of antiviral activities, sera containing antibodies immunoreactive to OvIFNτ (1-37), OvIFNτ (62-92), and OvIFNτ (139-172) were also the most effective inhibitors of OvIFNτ antiviral activity, with antibodies directed against the N-terminal and C-terminal peptides being the most efficacious.

The same sera were also used to examine their effect on the binding of IFNτ to its receptor.

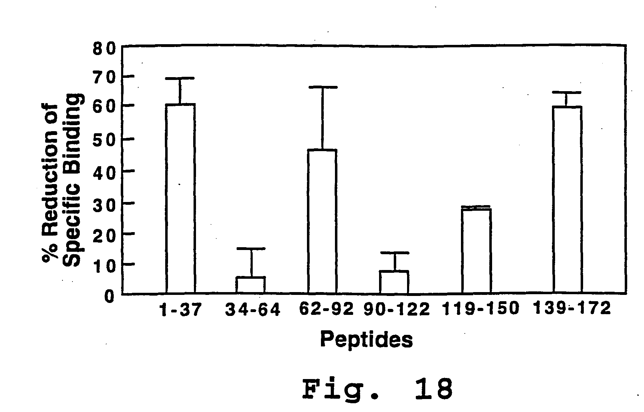

The IFNτ binding assay was carried out as follows. Five µg of IFNτ was iodinated for 2 minutes with 500 µCi of Na¹²⁵I (15 mCi/µg; Amersham Corporation, Arlington Heights, IL) in 25 µl of 0.5 M potassium phosphate buffer, pH 7.4, and 10 µl of chloramine-T (5 mg/ml) (Griggs, *et al.,* 1992). The specific activity of the iodinated protein was 137 µCi/µg. For binding assays, monolayers of MDBK cells were fixed with paraformaldehyde and blocked with 5% nonfat dry milk. Cells were incubated with 5 nM ¹²⁵I-IFNτ in phosphate buffered saline with 1% BSA for 2 hours at 4°C in the presence or absence of a 1:30 dilution of sera containing antibodies raised against IFNτ peptides or the appropriate preimmune sera. Specific binding was assessed by incubation with a 100-fold molar excess of unlabeled IFNτ. Specific binding of 36% was determined by competition with 500 nM unlabeled IFNτ. For example, total counts bound were 6850 ± 133, and a 100-fold molar excess of OvIFNτ produced 4398 ± 158 counts per minute. After incubation, the monolayers were washed three times, solubilized with 1% sodium dodecyl sulfate, and the radioactivity counted. Data from three replicate experiments are presented in Figure 18 as the mean percent reduction of OvIFNτ specific binding produced by antipeptide antisera relative to the appropriate preimmune sera ± standard deviation. Significant differences were assessed by analysis of variance followed by Scheffe's F test.

The same sera (containing antibodies immunoreactive to OvIFNτ (1-37), OvIFNτ (62-92), and OvIFNτ (139-172)) were the most effective inhibitors of ¹²⁵I-IFNτ binding to its receptor on MDBK cells. The lack of effect of sera immunoreactive with other IFNτ-derived peptides was not a function of titer against OvIFNτ, since each sera had equal or greater titer to their respective peptide relative to the three inhibiting sera: similar results were obtained when sera reactivity against the whole OvIFNτ molecule was assessed by ELISA for each sera.

These peptides, although apparently binding to the interferon receptor, did not in and of themselves elicit interferon-like effects in the cells.

### C. Anti-Proliferative Activity.

Functionally important sites for the antiproliferative activity of IFNτ were also examined using synthetic peptides (Table 11). Cellular proliferation was assayed as described above using MDBK cells. MDBK cells were cultured at 5 × 10⁵ cells/well in experiments 1 and 2 or 10 × 10⁵ cells in experiment 3 and treated with medium alone, IFNτ at a concentration of 300 units/ml and peptides at 1 mM for 48 hours. Duplicate wells were counted in each of three replicate experiments. For statistical analysis, data were normalized based on medium alone and assessed by analysis of variance followed by Least Significant Difference multiplate comparison test (p > 0.05).

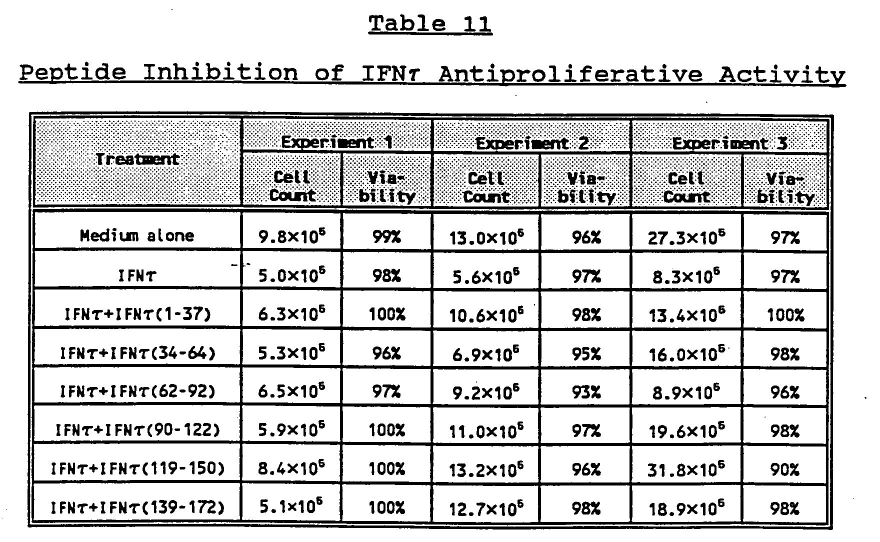

When proliferation of MDBK cells was monitored over a two-day period, cell number increased roughly 2-fold with greater than 95% viability. Addition of 300 units/ml of OvIFNτ entirely eliminated cell proliferation without a decrease in cell viability. Ovine IFNτ (119-150) was the most effective inhibitor of IFNτ antiproliferative activity.

Antisera to IFNτ (119-150), which inhibited binding of OvIFNτ to receptor, also reversed the OvIFNτ antiproliferative effect. Several other peptides, notably IFNτ (139-172), reversed the OvIFNτ antiproliferative effect, but to a lesser extent.

### EXAMPLE 18

### Further Analysis of the Cellular and Anti-Viral Effects of IFNτ

### A. HIV Anti-Viral Effects.

The antiviral effects of IFNτ against HIV were evaluated by treating human PBMC cells with various amounts of either recombinant ovine IFNτ (r-OvIFNτ) or recombinant human IFNα₂ at the time of infection with HIV. Drug was present throughout the experiment. At day 7 and day 14, p24 production was determined (by ELISA (Wang, *et al*., 1988, 1989) and compared to a zero drug control. The results of this analysis are presented in Table 12.

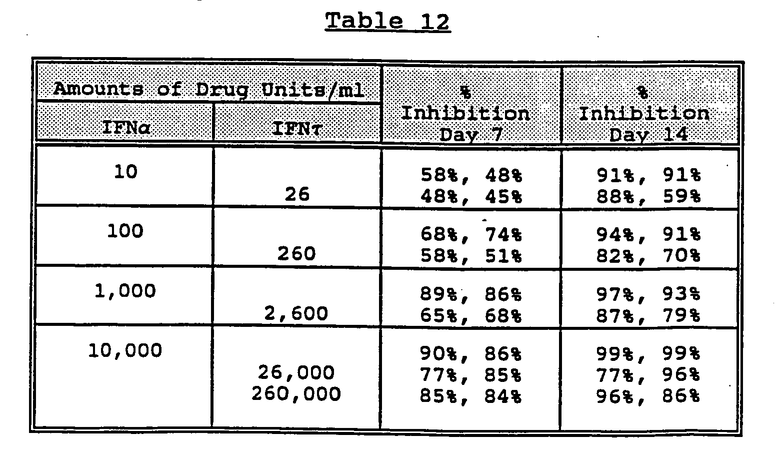

The data from these experiments support the conclusion that, at relatively low concentrations, IFNα and IFNτ are effective in reducing the replication of HIV in human lymphocytes.

### B. In vitro Cytotoxicity Test in PBMC's

Human PBMC's were seeded at 5 × 10⁵ cells/ml. Cells were stimulated at day 0 with 3 µg/ml PHA. Cells were treated with recombinant human IFNα2A (at concentrations of 10, 100, 1,000 and 10,000 units/ml) and IFNτ (at concentrations of 2.6, 26, 260, 2,600, 26,000, 260,000, and 2,600,000 units/ml) in 200 µl/wells (4 replicates of each concentration using 96 well flat bottom plates). Control cultures were given no interferons. After 4 days of incubation, cells were pulsed for 9 hours using ³H-thymidine at 1 uCi/well. The cells were harvested and the incorporation of labeled thymidine into DNA was determined (Figure 8).

No cytotoxicity was observed by measuring the uptake of thymidine at any concentration of IFNτ. However, rHuIFNα2 was toxic to cells at 1,000 units/ml.

In a second experiment, the same human PBMC's were treated with either IFNτ or human IFNα2A at concentrations of 100 units/ml or 10,000 units/ml. After 3 days or 8 days of incubation, viable cells were counted by flow cytometry. The results of this analysis are presented in Table 13.

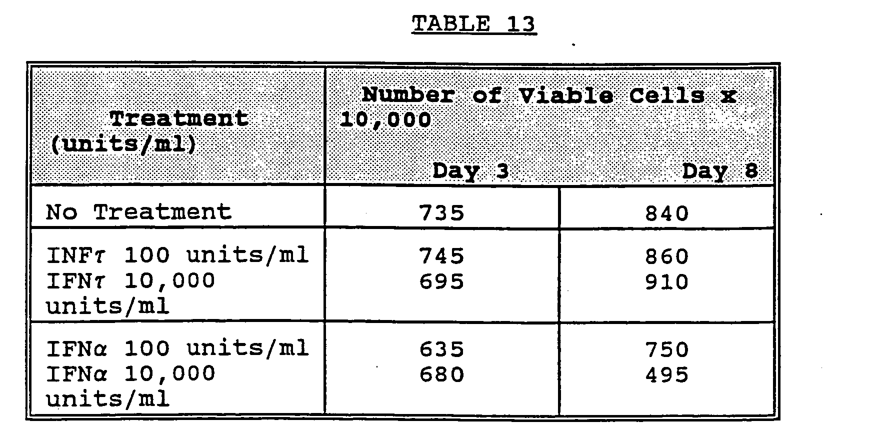

No cytotoxicity was observed in the cells treated with IFNτ. However, there was 10% cell death in IFNα treated cells at Day 3 and 49% cell death at Day 8.

### C. Inhibition of Hepatitis B Virus DNA Replication in Hepatocytes

The cell line used, HepG2-T14, is a human cell that was derived from liver cells transfected with Hepatitis B Virus (HBV). The cell line semi-stably produces HBV virus: over time the cell line's production of HBV intracellular DNA and secreted virus decreases. In order to maximize production of HBV DNA and virus, the cells are pre-treated with deAZA-C (5-azacytidine; Miyoshi, et al.) to induce production of the virus. Treatment was for 2-3 days and the amount of induction was about a factor of two.

The cells were then treated with either the IFNα and IFNτ at levels of 0, 5,000, 10,000, 20,000 and 40,000 units per ml.

All levels of either IFNα or IFNτ reduced DNA production by about a factor of 2 compared to the no drug control.

### D. Inhibition of Hepatospecific Messenger RNA Production in Hepatocytes

The hepatocyte cell line HepG2-T14 (described above) was examined for the effects of IFNα and IFNτ on hepatospecific mRNA production. Cells were incubated in concentrations of IFNα or IFNτ at 0, 5,000, 10,000, 20,000, and 40,000 units per ml. The messenger RNAs for the hepatocyte specific proteins Apo E and Apo A1 were detected by hybridization analysis (Sambrook, *et al.;* Maniatis, *et al.)* using probes specific for these two mRNA's (Shoulders, *et al.,* and Wallis, *et al*.).

No reduction of mRNA production was seen for Apo E or Apo A1 mRNA production with up to 40,000 units of either IFNα or IFNτ. This result suggests that the reduction of viral DNA replication in previous experiments was not due to the effects of IFNs on cellular house-keeping activities; rather the reduction was likely due to specific inhibition of viral replication in the host cells.

### EXAMPLE 19

### Isolation of Interferon-τ Fusion Protein

Sepharose 4B beads conjugated with anti-beta galactosidase is purchased from Promega. The beads are packed in 2 ml column and washed successively with phosphate-buffered saline with 0.02% sodium azide and 10 ml TX buffer (10 mM Tris buffer, pH 7.4, 1% aprotinin).

The IFNτ coding sequence (*e.g*., Figure 7) is cloned into the polylinker site of lambda gt11. The IFNτ coding sequence is placed in-frame with the amino terminal β-galactosidase coding sequences in lambda gt11. Lysogens infected with gt11/IFNτ are used to inoculate 500 ml of NZYDT broth. The culture is incubated at 32°C with aeration to an O.D. of about 0.2 to 0.4, then brought to 43°C quickly in a 43°C water bath for 15 minutes to induce gtl1 peptide synthesis, and incubated further at 37°C for 1 hour. The cells are pelleted by centrifugation, suspended in 10 ml of lysis buffer (10 mM Tris, pH 7.4 containing 2% "TRITON X-100" and 1% aprotinin added just before use.

The resuspended cells are frozen in liquid nitrogen then thawed, resulting in substantially complete cell lysis. The lysate is treated with DNaseI to digest bacterial and phage DNA, as evidenced by a gradual loss of viscosity in the lysate. Non-solubilized material is removed by centrifugation.

The clarified lysate material is loaded on the Sepharose column, the ends of the column closed, and the column placed on a rotary shaker for 2 hrs. at room temperature and 16 hours at 4°C. After the column settles, it is washed with 10 ml of TX buffer. The fused protein is eluted with 0.1 M carbonate/bicarbonate buffer, pH10. Typically, 14 ml of the elution buffer is passed through the column, and the fusion protein is eluted in the first 4-6 ml of eluate.

The eluate containing the fusion protein is concentrated in "CENTRICON-30" cartridges (Amicon, Danvers, Mass.). The final protein concentrate is resuspended in, for example, 400 µl PBS buffer. Protein purity is analyzed by SDS-PAGE.

For polyclonal antibodies, the purified fused protein is injected subcutaneously in Freund's adjuvant in a rabbit. Approximately 1 mg of fused protein is injected at days 0 and 21, and rabbit serum is typically collected at 6 and 8 weeks.

### EXAMPLE 20

### Preparation of Anti-IFNτ Antibody

### A. Expression of Glutathione-S-Transferase Fusion Proteins.

The IFNτ coding sequence (*e.g*., Figure 7) is cloned into the pGEX vector (Boyer, *et al*.; Frangioni, *et al*.; Guan, *et al*.; Hakes, *et al*.; Smith, *et al.,* 1988). The pGEX vector (Smith, *et al*.) was modified by insertion of a thrombin cleavage sequence in-frame with the glutathione-S-transferase protein (GST -- sj26 coding sequence). This vector is designated pGEXthr. The IFNτ coding sequence is placed in-frame with the sj26-thrombin coding sequences (Guan, *et al*.; Hakes, *et al*.). The IFNτ coding sequence insert can be generated by the polymerase chain reaction using PCR primers specific for the insert.

The IFNτ fragment is ligated to the linearized pGEXthr vector. The ligation mixture is transformed into *E. coli* and ampicillin resistant colonies are selected. Plasmids are isolated from the ampicillin resistant colonies and analyzed by restriction enzyme digestion to identify clones containing the IFNτ insert (vector designated pGEXthr-IFNτ).

*E. coli* strain XL-I Blue is transformed with pGEXthr-IFNτ and is grown at 37°C overnight. DNA is prepared from randomly-picked colonies. The presence of the insert coding sequence is typically confirmed by (i) restriction digest mapping, (ii) hybridization screening using labelled IFNτ probes (*i.e*., Southern analysis), or (iii) direct DNA sequence analysis.

### B. Partial Purification of Fusion Proteins.

A pGEXthr-IFNτ clone is grown overnight. The overnight culture is diluted 1:10 with LB medium containing ampicillin and grown for one hour at 37°C. Alternatively, the overnight culture is diluted 1:100 and grown to OD of 0.5-1.0 before addition of IPTG (isopropylthio-β-galactoside). IPTG (GIBCO-BRL, Gaithersburg MD) is added to a final concentration of 0.2-0.5 mM for the induction of protein expression and the incubation is typically continued for 2-5 hours, preferably 3.5 hours.

Bacterial cells are harvested by centrifugation and resuspended in 1/100 culture volume of MTPBS (150 mM NaCl, 16 mM Na₂HPO₄, 4 mM NaH₂PO₄). Cells are lysed by lysozyme, sonication or French press, and lysates cleared of cellular debris by centrifugation.

An aliquot of the supernatant obtained from IPTG-induced cultures of pGEXthr-IFNτ-containing cells and an aliquot of the supernatant obtained from IPTG-induced cultures of pGEXthr-vector alone are analyzed by SDS-polyacrylamide gel electrophoresis followed by Western blotting, as described below.

If necessary, the extracts can be concentrated by ultrafiltration using, for example, a "CENTRICON 10" filter.

Alternatively, the fusion proteins are partially purified over a glutathione agarose affinity column as described in detail by Smith, *et al*. In this method, 100 ml cultures are grown overnight. The cultures are diluted to 1 liter, and the cells grown another hour at 37°C. Expression of the fusion proteins is induced using IPTG. The induced cultures are grown at 37°C for 3.5 hours. Cells are harvested and a sonicator used to lyse the cells. Cellular debris is pelleted and the clear lysate loaded onto a glutathione "SEPHAROSE" column. The column is washed with several column volumes. The fusion protein is eluted from the affinity column with reduced glutathione and dialyzed. The IFNτ can be liberated from the hybrid protein by treatment with thrombin. The sj26 and IFNτ fragments of the hybrid protein can then be separated by size fractionation over columns or on gels.

Alternatively, the IFNτ portion of the hybrid protein is released from the column by treatment with thrombin (Guan, *et al*.; Hakes, *et al*.).

### C. Antibodies Against the Fusion Protein.

The purified Sj26/IFNτ fused protein is injected subcutaneously in Freund's adjuvant in a rabbit. Approximately 1 mg of fused protein is injected at days 0 and 21, and rabbit serum is typically collected at 6 and 8 weeks. A second rabbit is similarly immunized with purified Sj26 protein obtained from control bacterial lysate.

Minilysates from the following bacterial cultures are prepared: (1) KM392 cells infected with pGEXthr and pGEXthr containing the IFNτ insert; and (2) cells infected with lambda gtl1 containing the IFNτ insert. The minilysates and a commercial source β-galactosidase are fractionated by SDS-PAGE, and the bands transferred to nitrocellulose filters for Western blotting (Sambrook, *et al*.; Ausubel, *et al*.).

Summarizing the expected results, serum from control (Sj26) rabbits is immunoreactive with each of the Sj26 and Sj26 fused protein antigens. Serum from the animal immunized with Sj26/IFNτ fused protein is reactive with all Sj-26 and beta-gal fusion proteins containing IFNτ coding sequences, indicating the presence of specific immunoreaction with the IFNτ antigen. None of the sera are expected to be immunoreactive with beta-galactosidase.

Anti-IFNτ antibody present in the sera from the animal immunized with the Sj26/IFNτ is purified by affinity chromatography (using immobilized recombinantly produced IFNτ as ligand, essentially as described above in Example 12 for the anti-beta-galactosidase antibody).

While the invention has been described with reference to specific methods and embodiments, it will be appreciated that various modifications and changes may be made without departing from the invention.

## Claims

1. A composition for use in treating a disease responsive to treatment by interferon-α or by interferon-β in a subject comprising interferon-τ in an orally-administrable dosage form.

2. The composition according to claim 1, wherein said dosage form is a solid, semisolid or liquid dosage form.

3. The composition according to claim 1, wherein said dosage form is a tablet, a pill, a powder, a liquid solution or a liquid suspension.

4. The composition according to any one of claims 1-3 wherein said interferon-τ is administered at a dosage of at least about 1x10⁶ units/day.

5. Use of a composition according to any one of claims 1-4 for the manufacture of a medicament for treatment of a DNA or RNA viral infection.

6. Use of a composition according to any one of claims 1-4 for the manufacture of a medicament for inhibiting growth of a tumor.

7. A composition comprising interferon-τ in an orally-administrable dosage form for treating a disease responsive to treatmet by IFN-α or IFN-β.
